(19) Europäisches Patentamt
European Patent Office
Office européen des brevets

(11) Veröffentlichungsnummer: **0 033 720**
**B1**

(12) # EUROPÄISCHE PATENTSCHRIFT

(45) Veröffentlichungstag der Patentschrift: **07.03.84**

(21) Anmeldenummer: **81810025.7**

(22) Anmeldetag: **30.01.81**

(51) Int. Cl.³: **C 07 D 335/16,** C 08 F 28/06, C 08 G 63/18, C 08 G 69/42, C 08 F 2/50, G 03 C 1/68

(54) **Thioxanthoncarbonsäureester, -thioester und -amide, deren Verwendung als Sensibilisatoren oder Photoinitiatoren und Polymere.**

(30) Priorität: **05.02.80 CH 917/80**

(43) Veröffentlichungstag der Anmeldung: **12.08.81 Patentblatt 81/32**

(45) Bekanntmachung des Hinweises auf die Patenterteilung: **07.03.84 Patentblatt 84/10**

(84) Benannte Vertragsstaaten: **CH DE FR GB LI NL**

(56) Entgegenhaltungen: **CH - A - 564 550** **DE - B - 2 300 588**

(73) Patentinhaber: **CIBA-GEIGY AG, Postfach, CH-4002 Basel (CH)**

(72) Erfinder: **Kvita, Vratislav, Dr., Vogesenstrasse 71, CH-4153 Reinach (CH)**
Erfinder: **Zweifel, Hans, Dr., Lothringerstrasse 93, CH-4056 Basel (CH)**
Erfinder: **Roth, Martin, Dr., Chemin des Epinettes 12, CH-1723 Marly (CH)**
Erfinder: **Felder, Louis, Dr., Riehenring 5, CH-4058 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Thioxanthoncarbonsäureester, -thioester und -amide, deren Verwendung als Sensibilisatoren oder Photoinitiatoren und Polymere

Die Erfindung betrifft neue Thioxanthoncarbonsäureester, -thioester und -amide mit reaktiven funktionellen Gruppen, Verfahren zu deren Herstellung sowie deren Verwendung zur Herstellung von neuen Polymeren mit seitenständigen Thioxanthonresten. Die erfindungsgemässen Thioxanthonderivate sowie die daraus hergestellten Polymeren können als Sensibilisatoren für lichtvernetzbare Polymere oder als Initiatoren, gegebenenfalls im Gemisch mit Amiden, für die Photopolymerisation äthylenisch ungesättigter Verbindungen oder für die photochemische Vernetzung von Polyolefinen eingesetzt werden.

Aus der DE-A-2 811 755 ist es bekannt, dass sich gegebenenfalls halogenierte, besonders chlorierte, Thioxanthone als Sensibilisatoren für photoinduzierte Vernetzungsreaktionen eigenen. Voraussetzung für eine erfolgreiche derartige Anwendung ist eine gute Verträglichkeit des Sensibilisators im Polymeren, d.h. der Sensibilisator muss bis zu erhöhten Konzentrationen mit dem Polymeren mischbar sein. Ferner müssen die Sensibilisatoren in den bei der Verarbeitung der Polymeren zur Anwendung gelangenden Lösungsmitteln gut löslich sein. Die vorerwähnten bekannten Thioxanthone genügen diesen Anforderungen nicht in jeder Hinsicht; insbesondere entmischen sie sich leicht im Polymeren, wodurch dessen Sensibilisatorwirkung stark beeinträchtig wird.

Es wurden nun neue Thioxanthonderivate mit reaktiven funktionellen Gruppen gefunden, welche sich ausgezeichnet zur Verwendung als Sensibilisatoren für lichtvernetzbare Polymere und insbesondere zur Herstellung von Polymeren mit seitenständigen Thioxanthonresten eignen, die ihrerseits Anwendung als Sensibilisatoren für lichtvernetzbare Systeme finden. Die erfindungsgemässen (monomeren) Thioxanthonderivate zeichnen sich durch eine gute Verträglichkeit mit dem Polymeren und gute Löslichkeit in üblichen organischen Lösungsmitteln aus. Die daraus herstellbaren Polymeren haben vor allem den Vorteil einer wesentlich geringeren Entmischbarkeit mit den zu sensibilisierenden lichtvernetzbaren Systemen (geringere Migrationstendenz). Mit den neuen erfindungsgemässen Thioxanthonderivaten und den daraus herstellbaren Polymeren kann überdies überraschenderweise die UV-Absorption so beeinflusst werden, dass diese auch bei Bestrahlung mit langwelligem UV-Licht (bis 450 nm) eine sensibilisierende Wirkung ausüben und so die Vernetzung der photoempfindlichen Polymeren bewirken.

Es ist auch bekannt, dass man die Photopolymerisation von äthylenisch ungesättigten Verbindungen durch aromatische Ketone vom Typ des Benzophenons, des Anthrachinons, Xanthons und Thioxanthons initiieren kann. Es ist weiterhin aus der US Patentschrift 3 759 807 bekannt, dass die Initiatorwirkung solcher aromatischer Ketone durch den Zusatz von organischen Aminen beschleunigt werden kann. Da diese Amine allein meist keine Initiatorwirkung besitzen, wirken sie in Kombination mit aromatischen Ketonen als Aktivatoren oder Beschleuniger. Technisch ist dies von grosser Wichtigkeit, da die Produktionsgeschwindigkeit von photochemisch gehärteten Überzügen oder Druckfarben in erster Linie von der Polymerisationsgeschwindigkeit der ungesättigten Verbindung abhängt.

Gemische von erfindungsgemässen Thioxanthonderivaten oder den daraus herstellbaren Polymeren mit organischen Aminen als Initiatoren für die Photopolymerisation äthylenisch ungesättigter Verbindungen oder für die photochemische Vernetzung von Polyolefinen zeichnen sich durch eine hohe Polymerisationsgeschwindigkeit, eine geringe Vergilbungstendenz bei weiss pigmentierten Beschichtungen, teilweise durch eine gute Löslichkeit im Substrat und/oder eine hohe Lagerstabilität aus.

Die erfindungsgemässen Thioxanthoncarbonsäureester, -thioester und -amide mit reaktiven funktionellen Gruppen entsprechen der Formel I

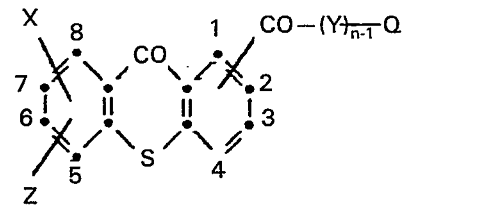

worin n die Zahl 1 oder 2, X Wasserstoff, Halogen, -CN, -OH, -SH, $-NO_2$, $-NH_2$, Phenylsulfonyl. Alkylsulfonyl, Alkyl, Alkoxy, Alkylthio, N,N-Dialkylamino oder -CO-Alkyl mit je 1-4 C-Atomen in den Alkylteilen, Z Wasserstoff, Halogen, -OH, -SH, Alkyl, Alkoxy, Alkylthio oder N,N-Dialkylamino mit je 1-4 C-Atomen in den Alkylteilen, Y $-OR_1-$, $-SR_1-$ oder $-N(R_2)R_1-$, $R_1$ gegebenenfalls verzweigtes Alkylen mit insgesamt 2-23 C-Atomen und 2-13 C-Atomen in der Hauptkette, Cyclopentylen, Cyclohexylen, Phenylen,

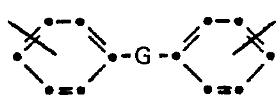 , 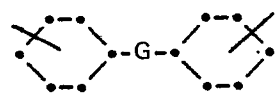

oder, wenn n = 2 und Q -OH oder $-OCO-C(R'')=$ $=CH_2$ sind, auch $-(CH_2CH_2O)_x-CH_2CH_2-$, $R_2$ Wasserstoff oder gegebenenfalls verzweigtes Alkyl mit insgesamt 1-23 C-Atomen und 1-13 C-Atomen in der Hauptkette, G $-CH_2-$, $-CH_2CH_2-$, $-C(CH_3)_2-$, -O-, -SO- oder -NH-, x eine ganze Zahl von 1-5, Q, wenn n = 1 ist, $-OCH_2CH\underset{O}{—}CH_2$, $-OCH_2-COOH$, $-OCH=$ $=CH_2$, $-OCH_2CH=CH_2$, $-SCH_2CH=CH_2$ oder $-NHCH_2CH=CH_2$ und wenn n = 2 ist, -OH, -SH, $-NH_2$, -NHR', $-SO_3H$, -COOH, -COCl, -NCO, -OCO-$-C(R'')=CH_2$, $-SCO-C(R'')=CH_2$, $-NHCO-C(R'')=$ $=CH_2$, $-OCH=CH_2$, $-OCH_2CH\underset{O}{—}CH_2$, -N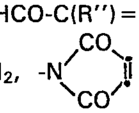 oder, wenn $R_1$ Alkylen oder Phenylen bedeutet, auch $-CH=CH_2$, R' Alkyl mit 1-5 C-Atomen und R'' Wasserstoff oder Methyl bedeuten. Verbindungen der Formel I, worin Q $-NH_2$ oder -NHR' bedeutet, können auch in Form von Salzen, besonders Salzen mit anorganischen Säuren, wie $H_2SO_4$ oder Salpetersäure und vor allem HCl, vorliegen.

Alkyl-, Alkoxy- oder Alkylthiogruppen X, Z, R' und $R_2$ bzw. Alkylteile in Resten X und Z können geradkettig oder verzweigt sein. Alkylengruppen $R_1$ und Alkylgruppen $R_2$ weisen bevorzugt insgesamt höchstens 18 und besonders höchstens 12 C-Atome auf.

Als Beispiele definitionsgemässer Alkyl-, Alkoxy-, Alkylthio-, Alkylsulfonyl-, N,N-Dialkylamino- und -CO-Alkylgruppen X, Z, R' oder $R_2$ seien genannt: die Methyl-, Äthyl-, n-Propyl-, Isopropyl-, n-Butyl-, sek.-Butyl-, n-Pentyl-, 2- oder 3-Pentyl-, n-Hexyl-, n-Heptyl-, 2- oder 3-Heptyl-, n-Octyl-, n-Nonyl-, n-Decyl-, 2-Decyl-, n-Dodecyl-, n-Tridecyl-, Tridec-7-yl-, Heptadec-9-yl, 2,6,10-Trimethyldodecyl- und 2,6,10,14-Tetramethylhexadecylgruppen; die Methoxy-, Äthoxy-, n-Propoxy-, Isopropoxy- und n-Butoxygruppe; die Methylthio-, Äthylthio- und n-Propylthiogruppe; die Methyl- und Äthylsulfonylgruppe; die N,N-Dimethylamino-, N,N-Diäthylamino-, N-Methyl-N-äthylamino- und N,N-Di-n-propylaminogruppe; die Acetyl-, Propionyl- und Butyrylgruppe.

Alkylgruppen R', X und Z sind bevorzugt geradkettig und weisen insbesondere 1 oder 2 C-Atomen auf.

Als Beispiele definitionsgemässer Alkylengruppen $R_1$ seien erwähnt: die 1,2-Äthylen-, Propylen-, Tetramethylen-, Isobutylen-, Pentamethylen-, Iso- und Neopentylen-, Hexamethylen-, Heptamethylen-, 2- oder 3-Methylhexylen-, Octamethylen-, Nonamethylen-, Decamethylen-, 2-Methyl-nonylen-, Dodecamethylen-, Tridecamethylen-, Hexylheptylen-, Octylnonylen-, 2,6,10-Trimethyldecylen-, 2,6,10,14-Tetramethyl-dodecylengruppe.

Stellt $R_1$ eine Cyclopentylen-, Cyclohexylen- oder Phenylengruppe dar, so handelt es sich z.B. um die 1,3-Cyclopentylen-, 1,3- oder insbesondere 1,4-Cyclohexylen-, 1,3- oder 1,4-Phenylengruppe. Als bicyclische Reste $R_1$ werden solche der Formeln

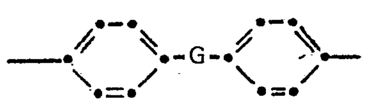 , 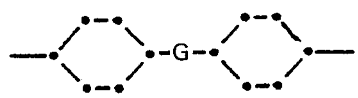

bevorzugt, worin G $-CH_2-$, -O- oder $-SO_2-$ bedeutet.

$R_1$ stellt bevorzugt Alkylen mit insgesamt 2-18 und insbesondere 2-12 C-Atomen, 1,3-Cyclopentylen, 1,4-Cyclohexylen, $-CH_2CH_2OCH_2CH_2-$ oder $-(CH_2CH_2O)_2-CH_2CH_2-$ dar. $R_2$ ist vorzugsweise Wasserstoff.

Bevorzugte Bedeutungen von Q bei n = 1 sind -OCH$_2$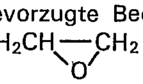 oder $-OCH=CH_2$ und bei n = 2 -OH, $-NH_2$, $-NHCH_3$, -COOH, -COCl, $-OCO-C(R'')=CH_2$, $-OCH=CH_2$, -OCH$_2$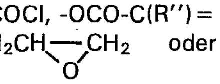 oder 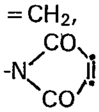 .

Unter den Verbindungen der Formel I sind einerseits solche bevorzugt, worin Z Wasserstoff bedeutet und X, Y, Q und n die unter Formel I angegebene Bedeutung haben. Die Gruppierung $-CO-(Y)_{n-1}-Q$ ist vorzugsweise in 1- oder 3-Stellung gebunden.

Eine weitere Klasse bevorzugter Verbindungen der Formel I sind solche, worin X in 6-Stellung gebunden ist und $-NO_2$, Alkylsulfonyl mit 1-4 und vor allem 1 oder 2 C-Atomen oder Phenylsulfonyl bedeutet, Z in 7-Stellung gebunden ist und Alkyl, Alkoxy, Alkylthio oder N,N-Dialkylamino mit je 1-4 und insbesondere 1 oder 2 C-Atomen in den Alkylteilen bedeutet und sich die Gruppierung $-CO-(Y)_{n-1}-Q$ in 1- oder 3-Stellung befindet.

Gemäss einer weiteren Bevorzugung stellt Z Wasserstoff dar, X ist in 6-Stellung gebunden und bedeutet $-NO_2$, Alkylsulfonyl mit 1-4 und insbesondere 1 oder 2 C-Atomen oder Phenylsulfonyl. Dabei ist die Gruppierung $-CO-(Y)_{n-1}-Q$ ebenfalls bevorzugt in 1- oder 3-Stellung gebunden.

Ganz besonders bevorzugt sind Verbindungen der Formel I, worin X Wasserstoff und Z Wasserstoff oder in 7-Stellung gebundenes Methyl oder Methoxy bedeutet und sich die Gruppe $-CO-(Y)_{n-1}-Q$ in 1- oder 3-Stellung befindet, vor allem solche, worin n = 1 ist und Q -OCH$_2$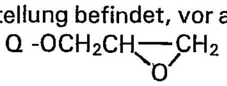 oder $-OCH=CH_2$ darstellt, oder worin n = 2 ist, Y $-OR_1-$ oder $-NHR_1-$, $R_1$ Alkylen mit 2-6 C-Atomen oder bei Q = -OH oder $-OCOC(R'')=CH_2$ auch $-CH_2CH_2OCH_2CH_2-$ oder $-(CH_2CH_2O)_2CH_2CH_2-$ und Q -OH, $-NH_2$, $-NHCH_3$, -COOH, -COCl, $-OCO-C(R'')=CH_2$, $-OCH=CH_2$, -OCH$_2$-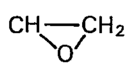 oder 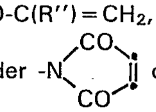 darstellen, sowie Salze von derartigen bevorzugten Verbindungen der Formel I, worin Q $-NH_2$ oder $-NHCH_3$ ist, mit anorganischen Säuren. Besonders bevorzugt stellt Q bei n = 1 $-OCH=CH_2$ und bei n = 2 -OH, $-NH_2$, $-OCH=CH_2$ oder $-OCO-C(R'')=CH_2$ dar, wobei, wenn Q $-NH_2$ darstellt, Salze mit HCl bevorzugt sind.

Die Verbindungen der Formel I und deren definitionsgemässe Salze können dadurch hergestellt werden, dass man

a) eine Verbindung der Formel II

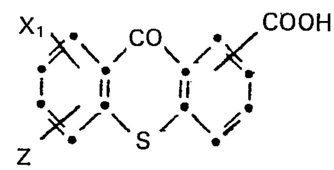

(II)

einen $C_{1-6}$-Alkylester einer Verbindung der Formel II oder ein Säurechlorid einer Verbindung der Formel II mit Z und $X_1$ ungleich -SH oder -OH mit einer Verbindung der Formel IIIa

$$H(Y)_{n-1}Q^1 \qquad \text{(IIIa)}$$

oder, bei Q' = $-NH_2$ oder -NHR', einem Salz einer Verbindung der Formel IIIa zu einer Verbindung der Formel Ia

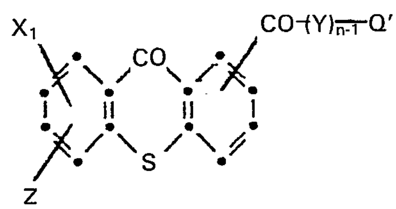

(Ia)

bzw. entsprechenden Salzen umsetzt,

b) eine Verbindung der Formel II oder einen $C_{1-6}$-Alkylester einer Verbindung der Formel II mit einer Verbindung der Formel IIIb

$CH_2=CH\text{-}OCO\text{-}R'$ (IIIb)

zu einer Verbindung der Formel Ib

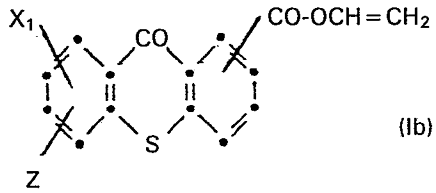

(Ib)

umsetzt, oder

c) ein Säurechlorid einer Verbindung der Formel II zuerst mit einem Salz einer Verbindung der Formel IIIc

$H\text{-}Y\text{-}NH_2$ (IIIc)

zum entsprechenden Salz einer Verbindung der Formel IV

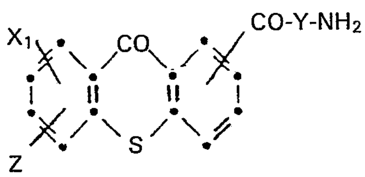

(IV)

umsetzt und das Salz einer Verbindung der Formel IV in Gegenwart eines inerten organischen Lösungsmittels mit Phosgen zu einer Verbindung der Formel Ic

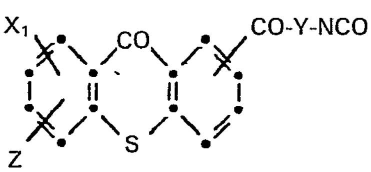

(Ic)

umsetzt, wobei Y, Z, R' und n die unter Formel I angegebene Bedeutung haben, $X_1$ Wasserstoff, Halogen, -CN, -OH, -SH, $-NO_2$, Phenylsulfonyl, Alkylsulfonyl, Alkyl, Alkoxy, Alkylthio, N,N-Dialkylamino oder -CO-Alkyl mit je 1-4 C-Atomen in den Alkylteilen darstellt,

Q', bei n = 1, 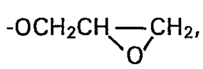 $-OCH_2\text{-}COOH$, $-OCH_2CH=CH_2$, $-SCH_2CH=CH_2$ oder $-NHCH_2CH=CH_2$ und bei n = 2, -OH, -SH, $-NH_2$, -NHR', $-SO_3H$, -COOH, $-OCO\text{-}C(R'')=CH_2$, $-SCO\text{-}C(R'')=CH_2$, $-NHCO\text{-}C(R'')=CH_2$, 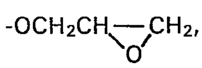 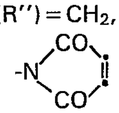

oder, wenn $R_1$ Alkylen oder Phenylen bedeutet, auch $-CH=CH_2$ und R'' Wasserstoff oder Methyl darstellen, und gegebenenfalls anschliessend die Gruppe $X_1$ in den Verbindungen der Formeln Ia, Ib oder Ic in eine davon verschiedene Gruppe X und/oder die Gruppe Q' = -COOH in Formel Ia durch Behandlung mit geeigneten Chlorierungsmitteln in die Gruppe -COCl überführt. Als Salze von Verbindungen der Formeln IIIa, IIIc, Ia (Q' = $-NH_2$ oder -NHR') und IV kommen insbesondere Salze mit anorganischen Säuren, wie $H_2SO_4$ oder Salpetersäure und vor allem HCl, in Betracht. Salze von Verbindungen der Formel Ia mit Q' = $-NH_2$ oder -NHR' können gewünschtenfalls auf an sich bekannte Weise durch Zusatz von geeigneten Basen in die entsprechenden Amine übergeführt werden.

Zur allfälligen Überführung in die Säurechloride eignen sich nur Verbindungen der Formel II, worin $X_1$ und Z ungleich -OH oder -SH sind. Als Chlorierungsmittel können z.B. Thionylchlorid, $PCl_5$ oder Oxalylchlorid verwendet werden. Definitionsgemässe Alkylester von Verbindungen der Formel II werden zweckmässig aus den entsprechenden Säurechloriden hergestellt.

Die obigen Umsetzungen können auf an sich bekannte Weise und je nach Art der Reaktionskomponenten mit oder ohne Zusatz inerter organischer Lösungsmittel, wie Dioxan, Benzol, Toluol, Methylenchlorid oder Chloroform, durchgeführt werden. Säurechloride von Verbindungen der Formel II werden mit Alkoholen oder Thiolen der Formel IIIa im allgemeinen bei Temperaturen zwischen etwa 25-80°C umgesetzt. Als Lösungsmittel verwendet man zweckmässig einen Überschuss des entsprechenden Alkohols oder Thiols. Die Umsetzung der Säurechloride von Verbindungen der Formel II mit Aminen der Formel IIIa erfolgt mit Vorteil bei Temperaturen zwischen etwa 0 und 40°C. Die Umsetzung der freien Säuren der Formel II mit Alkoholen der Formel IIIa wird zweckmässig unter azeotroper Wasserabscheidung und unter Zusatz katalytischer Mengen Säure, wie $H_2SO_4$ oder p-Toluolsulfonsäure, oder in Gegenwart wasserabspaltender Mittel, wie HCl-Gas oder konzentrierter Schwefelsäure, durchgeführt. Als Schleppmittel verwendet man dabei bevorzugt Benzol, Toluol oder Chloroform. Die Umesterung von Alkylestern von Verbindungen der Formel II erfolgt mit Vorteil unter Zusatz von Säuren, wie HCl oder $H_2SO_4$, Aluminiumalkoholaten, basischen oder sauren Ionenaustauschern. Die Umsetzung von Verbindungen der Formel II oder deren Alkylestern mit Verbindungen der Formel IIIb wird zweckmässig in Gegenwart von Katalysatoren, wie $HgCl_2$, $Li_2(PdCl_4)$ oder $PdCl_2$, vorgenommen.

Einige Verbindungen der Formel I können auch nach abgeänderten Verfahren, z.B. wie folgt hergestellt werden:

1). Verbindungen der Formel I, worin Y $-OR_1-$, Q bei n = 1, 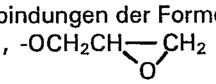 oder $-OCH_2CH=CH_2$ und bei n = 2, -OH, -SH, $-NH_2$, -NHR', $-OCO\text{-}C(R'')=CH_2$, $-NHCO\text{-}C(R'')=CH_2$, $-SCO\text{-}C(R'')=CH_2$, $-CH=CH_2$, $-OCH=CH_2$ oder 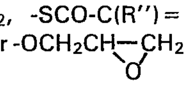

darstellt und X, Y und Z die unter Formel I angegebene Bedeutung haben, durch Umsetzung eines Alkalimetall- oder Erdalkalimetallsalzes einer Verbindung

der Formel II mit einem Halogenid der Formel V

$$Hal\text{-}(R_1)_{\overline{n-1}}Q'' \qquad (V)$$

gegebenenfalls in Gegenwart einer Base, wobei Hal ein Halogenatom, besonders Chlor oder Brom, und Q'' bei n = 1 $-CH_2\underset{O}{CH-CH_2}$ oder $-CH_2CH=CH_2$ und bei n = 2 -OH, -SH, $-NH_2$, -NHR', -OCO-C(R'') = $=CH_2$, -NHCO-C(R'') = $CH_2$, -SCO-C(R'') = $CH_2$, $-CH=CH_2$, $-OCH=CH_2$ oder $-OCH_2\underset{O}{CH-CH_2}$ bedeuten und $R_1$ und n die oben angegebene Bedeutung haben, und gegebenenfalls anschliessende Überführung der Gruppe $X_1$ in eine davon verschiedene Gruppe X.

2). Verbindungen der Formel I, worin X, Y und Z die angegebene Bedeutung haben, X und Z aber ungleich -OH, -SH oder $-NH_2$ sind, n die Zahl 2 und Q 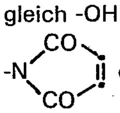 darstellen, durch Umsetzung einer Verbindung der Formel IV, worin $X_1$ und Z ungleich -OH, -SH oder $-NH_2$ sind, mit Maleinsäureanhydrid, Cyclisierung der erhaltenen Amidcarbonsäure in an sich bekannter Weise, z.B. in Gegenwart von Acetanhydrid und Natriumacetat, und gegebenenfalls anschliessende Überführung der Gruppe $X_1$ in eine davon verschiedene Gruppe X.

3). Durch Umsetzung von Verbindungen der Formel I, worin X, Y und Z die angegebene Bedeutung haben, X und Z jedoch ungleich -OH, -SH oder $-NH_2$ sind, n die Zahl 2 und Q -OH, -SH oder $-NH_2$ bedeuten, mit einem Halogenid der Formel VI

$$Hal\text{-}CO\text{-}C(R'')=CH_2 \qquad (VI)$$

worin Hal ein Halogenatom, besonders Chlor oder Brom bedeutet und R'' die unter Formel I angegebene Bedeutung hat.

4). Verbindungen der Formel I mit n = 2 oder 1 und Q = $-OCH_2\underset{O}{CH-CH_2}$ durch Umsetzung eines Alkalimetall- oder Erdalkalimetallsalzes einer Verbindung der Formel Id

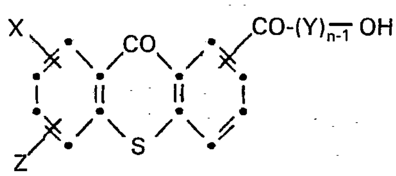

(Id)

mit Epichlorhydrin oder Epibromhydrin, gegebenenfalls in Gegenwart einer Base, wobei n, X, Y und Z die unter Formel I angegebene Bedeutung haben.

Als Alkalimetall- oder Erdalkalimetallsalze von Verbindungen der Formel II bzw. Id setzt man bei den obigen Reaktionen z.B. Calcium- oder Bariumsalze und insbesondere Natrium- oder Kaliumsalze ein. Als Basen können z.B. Amine, wie Diäthylamin und Triäthylamin, eingesetzt werden.

Die Umsetzungen 1) und 4) können auch mittels Phasentransfer-Katalyse vorgenommen werden, z.B. in Gegenwart von Tetraalkyl- oder Trialkylbenzylammoniumhalogeniden mit je 1-4 C-Atomen in den Alkylteilen, und $K_2CO_3$ oder $Na_2CO_3$.

Die Ausgangsverbindungen der Formel IIIa, IIIb und IIIc sind bekannt oder können nach an sich bekannten Methoden hergestellt werden. Die Verbindungen der Formel II sind teilweise ebenfalls bekannt. Sie können auf an sich bekannte Weise hergestellt werden [vgl. deutsche Offenlegungsschrift 2 344 799 und US Patentschrift 4 101 558], z.B. durch Cyclisieren einer Verbindung der Formel VIIa oder VIIb

(VIIa) 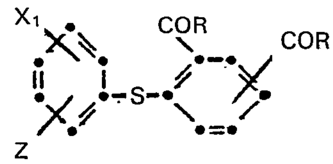

oder

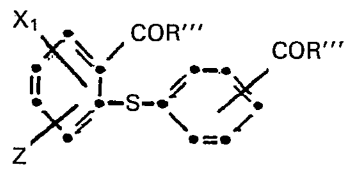

(VIIb)

worin $X_1$ und Z die oben angegebene Bedeutung haben und R und R''' -OH oder, wenn Z und $X_1$ ungleich -OH oder -SH sind, auch Chlor bedeuten, oder, wenn sich die Gruppen -COR in ortho-Stellung zueinander befinden, die beiden R zusammen -O- darstellen, unter gleichzeitiger Hydrolyse von Chloratomen R oder R'''.

Ausgangsprodukte der Formel II, worin $X_1$ Wasserstoff, Halogen, -CN, $-NO_2$, Phenylsulfonyl, Alkylsulfonyl, Alkyl, Alkoxy, Alkylthio oder N,N-Dialkylamino mit je 1-4 C-Atomen in den Alkylteilen und Z Wasserstoff, Halogen, Alkyl, Alkoxy, Alkylthio oder N,N-Dialkylamino mit je 1-4 C-Atomen in den Alkylteilen bedeuten, können auch dadurch hergestellt werden, dass man

α) eine Verbindung der Formel VIIIa

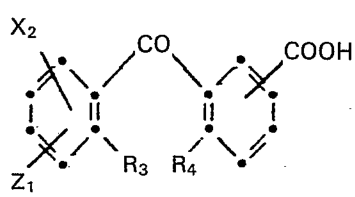

(VIIIa)

worin $Z_1$ Wasserstoff, Halogen, Alkyl, Alkoxy, Alkylthio oder N,N-Dialkylamino mit je 1-4 C-Atomen in den Alkylteilen, $X_2$ Wasserstoff, Halogen, -CN, $-NO_2$, Phenylsulfonyl, Alkylsulfonyl, Alkyl, Alkoxy, Alkylthio oder N,N-Dialkyllamino mit je 1-4 C-Atomen in den Alkylteilen, eines von $R_3$ und $R_4$ eine Mercaptogruppe und das andere eine abspaltbare Gruppe bedeuten, cyclisiert oder

β) eine Verbindung der Formel VIIIb

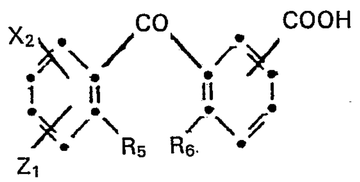

(VIIIb)

mit einem anorganischen Sulfid zu einer Verbindung der Formel II cyclisiert, wobei $X_2$ und $Z_1$ die oben angegebene Bedeutung haben und $R_5$ und $R_6$ unabhängig voneinander eine abspaltbare Gruppe darstellen.

Schliesslich können Ausgangsverbindungen der Formel II, worin $X_1$ Wasserstoff, Halogen, $-NO_2$, Phenylsulfonyl, Alkylsulfonyl, Alkyl, Alkoxy, Alkylthio, N,N-Dialkylamino oder -CO-Alkyl mit je 1-4 C-Atomen in den Alkylteilen und Z Wasserstoff, Halogen, Alkyl, Alkoxy, Alkylthio oder N,N-Dialkylamino mit je 1-4 C-Atomen in den Alkylteilen bedeuten, auch dadurch erhalten werden, dass man eine Verbindung der Formel IX

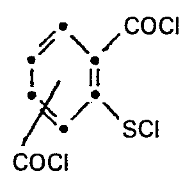

(IX)

in Gegenwart eines Friedel-Crafts-Katalysators mit einer Verbindung der Formel X

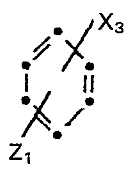

(X)

umsetzt, und den enstandenen Komplex zu einer Verbindung der Formel II zersetzt, wobei $X_3$ Wasserstoff, Halogen, $-NO_2$, Phenylsulfonyl, Alkylsulfonyl, Alkyl, Alkoxy, Alkylthio, N,N-Dialkylamino oder -CO-Alkyl mit je 1-4 C-Atomen in den Alkylteilen bedeutet und Z, die oben angegebene Bedeutung hat.

Die Cyclisierung der Verbindungen der Formel VIIa und VIIb wird mit Vorteil in Gegenwart einer Protonensäure oder einer Lewis-Säure vorgenommen. Beispiele geeigneter Protonensäuren sind Polyphosphorsäure, gegebenenfalls im Gemisch mit Phosphoroxichlorid, Chlorsulfonsäure und Schwefelsäure. Geeignete Lewis-Säuren sind z.B. Aluminiumtrichlorid oder Bortrifluorid.

Die Ausgangsverbindungen der Formel VIIa und VIIb lassen sich z.B. analog den in der deutschen Offenlegungsschrift 2 344 799 beschriebenen Verfahren durch Umsetzung von geeignet substituierten Thiophenolen oder Derivaten davon, wie Alkalimetall- oder Erdalkalimetallsalzen, mit Nitro- oder Halogenbenzolen herstellen. Dabei müssen das Thiophenol und das Nitro- oder Halogenbenzol zusammen mindestens zwei Gruppen -COR bzw. -COR''' oder zwei in Gruppen -COR oder -COR''' überführbare Gruppen, wie Nitrilgruppen, aufweisen, wovon sich eine in ortho-Stellung zur SH-Gruppe bzw. zur Nitrogruppe oder zu einem Halogenatom befinden muss.

Geeignete Nitrobenzole sind solche, die neben der Nitrogruppe noch eine oder mehrere elektronenanziehende Gruppen, wie Carbonsäureester-, Carbonsäurechlorid-, Nitril-, Anhydrid- oder Imidgruppen aufweisen. Nach einem bevorzugten Verfahren werden Verbindungen Formel VIIa durch Umsetzung eines Nitrophthal-, -isophthal- oder -terephthalsäuredicarbonsäurealkylesters mit einem geeignet substituierten Thiophenol und anschliessende Verseifung des erhaltenen Dicarbonsäurealkylesters einer Verbindung der Formel VIIa hergestellt.

Als abspaltbare Gruppen $R_3$ bis $R_6$ kommen vor allem Halogenatome, Nitro-, Arylsulfonyl- und Sulfinylgruppen in Betracht. Bevorzugte abspaltbare Gruppen $R_3$ bis $R_6$ sind Halogenatome, besonders Chlor, und Nitrogruppen.

Als anorganisches Sulfid zur Umsetzung mit Verbindungen der Formel VIIIb verwendet man mit Vorteil Alkalimetall- oder Erdalkalimetallsulfide oder -hydrosulfide, bevorzugt Natriumsulfid. Die Ausgangsprodukte der Formel VIIIa und VIIIb können auf an sich bekannte Weise durch Friedel-Crafts-Reaktion von entsprechend substituierten Acylhalogeniden mit geeignet substituierten nucleophilen aromatischen Verbindungen erhalten werden.

Die Kondensation der Verbindungen der Formel IX mit den Verbindungen der Formel X in Gegenwart von Friedel-Crafts-Katalysatoren, besonders Aluminiumtrichlorid, wird zweckmässig in organischem Medium bei Temperaturen zwischen etwa 10 und 80°C durchgeführt. Nach Beendigung der Umsetzung kann der entstandene Komplex durch Eingiessen in ein Wasser/Eis-Gemisch oder durch Zugabe von verdünnter Mineralsäure, wie Salzsäure, oder von wässrigen Alkalimetall- oder Erdalkalimetallhydroxid-Lösungen, wie Natrium-, Kalium-, Barium- oder Calciumhydroxid, zersetzt werden.

Die Überführung von Gruppen $X_1$, $X_2$ oder $X_3$ in Gruppen X kann auf an sich bekannte Weise vorgenommen werden. So lassen sich beispielsweise Nitrogruppen $X_1$, $X_2$ oder $X_3$ nach an sich bekannten Methoden zu Aminogruppen reduzieren, die ihrerseits in Halogenatome, -OH, -SH, -CN, Alkoxy- oder N,N-Dialkylaminogruppen übergeführt werden können. Cyanogruppen $X_1$ oder $X_2$ lassen sich in Gruppen X = -CO-Alkyl umwandeln. Durch Alkylsulfonyl- oder Phenylsulfonylgruppen substituierte Thioxanthone der Formel I lassen sich z.B. durch Umsetzung der entsprechenden Nitroverbindungen mit Alkalimetall-alkyl- oder -phenylsulfinaten herstellen.

Dank der reaktiven funktionellen Gruppen Q eignen sich die erfindungsgemässen Thioxanthonderivate der Formel I besonders zur Herstellung von Polymeren, die ihrerseits Anwendung als Sensibilisatoren für lichtvernetzbare Systeme (Polymere) oder als Photoinitiatoren finden. Gegenstand der Erfindung sind somit auch Polymere, deren Durchschnittsmolekulargewicht mindestens 1000 beträgt und die seitenständige Thioxanthongruppierungen der Formel I

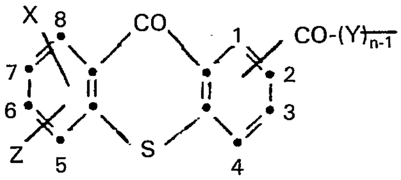

(I')

aufweisen, worin X, Y, Z und n die unter Formel I angegebene Bedeutung haben und wobei der Anteil an Gruppierungen der Formel I' mindestens 2 Prozent, bevorzugt 2-100 und insbesondere 20-100 Prozent, bezogen auf die Anzahl der wiederkehrenden Strukturelemente des Polymeren, beträgt.

Die erfindungsgemässen Polymeren weisen zweckmässig ein Durchschnittsmolekulargewicht von mindestens 1000 bis 500000 und insbesondere ein Durchschnittsmolekulargewicht von etwa 1000 bis 100000 auf.

Das Durchschnittmolekulargewicht wird nach an sich bekannten Methoden, z.B. mittels Streulichtbestimmung oder Gelpermeationschromatographie, bestimmt.

Bei den erfindungsgemässen Polymeren handelt es sich z.B. um Polyäther, Polyamine, Polyimine, Polykondensate auf der Basis von Phenol-Formaldehyd, Polysaccharide, Gelatine und vor allem um Polymere, welche durch Homo- oder Copolymerisation von C = C-Doppelbindungen enthaltenden Monomeren erhalten werden.

Die erfindungsgemässen Polymeren lassen sich nach an sich bekannten Synthesemethoden zur Herstellung von Makromolekülen mit seitenständigen Gruppen herstellen. Grundsätzlich kommen folgende Wege in Betracht:

1. Einbau der Thioxanthonreste der Formel I' in eine bestehende Polymerkette;

2. Aufbau der Polymerkette aus Monomeren, welche die Thioxanthongruppierung der Formel I' bereits enthalten, wobei die Polymerkette durch Polymerisation oder Polyaddition aufgebaut werden kann.

Teilweise können mit den Methoden 1 und 2 gleiche Produkte erhalten werden, so dass je nach Art der funktionellen Gruppen wahlweise die Methode 1 oder 2 anwenden lässt. Werden die Thioxanthonreste in eine bereits vorhandene Polymerkette eingebaut, so erfolgt dieser Einbau entweder durch eine Kondensationsreaktion oder durch eine Additionsreaktion unter gleichzeitiger Öffnung eines Ringsystems, z.B. einer Dicarbonsäureanhydridgruppe oder einer Epoxidgruppe.

Nach der vorerwähnten Aufbau-Methode können erfindungsgemässe Polymere dadurch herstellt werden, dass man eine Verbindung der Formel Ie

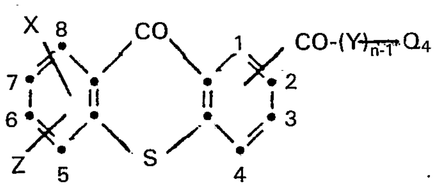

(Ie)

worin $Q_4$ bei n = 1, $-OCH=CH_2$, $-OCH_2CH=CH_2$, $-SCH_2CH=CH_2$ oder $-NHCH_2CH=CH_2$, und bei n = 2 $-OCO-C(R'')=CH_2$, $-SCO-C(R'')=CH_2$, $-NHCO-C(R'')=CH_2$, $-OCH=CH_2$, 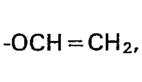 oder, wenn $R_1$ Alkylen oder Phenylen bedeutet, auch $-CH=CH_2$ darstellt und X, Y, Z, R'' und n die unter Formel I angegebene Bedeutung haben, gegebenenfalls in Gegenwart von Comonomeren zur Reaktion bringt, wobei das Molverhältnis von Verbindung der Formel Ie zu Comonomeren 1:49 bis 1:0 beträgt.

$Q_4$ stellt dabei bevorzugt 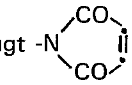, $-OCH=CH_2$ oder $-OCO-C(R'')=CH_2$ dar.

Nach der vorerwähnten Einbau-Methode lassen sich erfindungsgemässe Polymere zum Beispiel dadurch herstellen, dass man eine Verbindung der Formel If

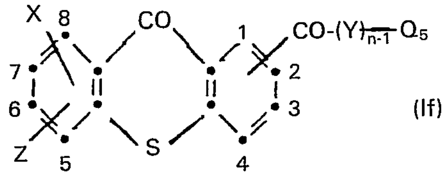

(If)

worin X' Wasserstoff, Halogen, -CN, $-NO_2$, Phenylsulfonyl, Alkylsulfonyl, Alkyl, Alkoxy, Alkylthio, N,N-Dialkylamino oder -CO-Alkyl mit je 1-4 C-Atomen in den Alkylteilen, $Z_1$ Wasserstoff, Halogen, Alkyl, Alkoxy, Alkylthio oder N,N-Dialkylamino mit je 1-4 C-Atomen in den Alkylteilen und $Q_5$ bei n = 1, $-OCH_2\underset{O}{CH—CH_2}$ oder $-OCH_2-COOH$ und bei n = 2 -OH, -SH, $-NH_2$, -NHR', $-SO_3H$, -COOH, -COCl, -NCO oder $-OCH_2\underset{O}{CH—CH_2}$ darstellen und Y, R' und n die unter Formel I angegebene Bedeutung haben, oder Salze von Verbindungen der Formel If, worin $Q_5$-$NH_2$ oder -NHR' darstellt, mit einem Polymeren mit entsprechenden funktionellen Gruppen in einem Verhältnis von 1:50 bis 1:1, bezogen auf die Anzahl der wiederkehrenden Strukturelemente des Polymeren, zur Reaktion bringt.

Verbindungen der Formel If, worin $Q_5$ -COOH, -COCl, $-SO_3H$ oder $-OCH_2COOH$ bedeutet, lassen sich beispielsweise mit Polymeren umsetzen, die freie OH-, $NH_2$-, NH-Alkyl- oder SH-Gruppen aufweisen. Verbindungen der Formel If, worin $Q_5$ -OH, -SH, $-NH_2$, -NHR', -NCO oder $-OCH_2\underset{O}{CH—CH_2}$ bedeutet, eignen sich z.B. zur Umsetzung mit Polymeren, die Anhydrid- bzw. -COOH-Gruppen aufweisen. Schliesslich können Verbindungen der Formel If, worin $Q_5$ -OH, -SH, $-NH_2$, -NHR' oder -COOH bedeutet, auch mit Polymeren umgesetzt werden, die $-CH_2\underset{O}{CH—CH_2}$-Gruppen aufweisen.

Verbindungen der Formel Ie eignen sich zur Homopolymerisation oder zur Copolymerisation mit anderen äthylenisch ungesättigten Comonomeren, insbesondere solchen der weiter unten angegebenen Art.

Als Beispiele von Ausgangspolymeren, die sich mit Verbindungen der Formel If umsetzen lassen, seien erwähnt: Polyacrylsäure, Polymethacrylsäure, Copolymere aus diesen Säuren und anderen äthylenisch ungesättigten Monomeren, Copolymere aufgebaut aus Maleinsäureanhydrid und äthylenisch ungesättigten Monomeren, wie Methylvinyläther, Äthylen, Styrol, Hexen-1, Decen-1, Tetradecen-1 und Octadecen-1, Polymere mit freien Hydroxylgruppen, wie Homo- und Copolymere von Acrylsäure- und Methacrylsäurehydroxyalkylestern, Polyvinylalkohole, native oder regenerierte Cellulose, Cellulosederivate, Hydroxyalkylcellulose, Polyäther mit freien -OH-Gruppen, Phenol-Formaldehyd-Polykondensate, Polymere mit freien Glycidylgruppen, wie Copolymere auf der Basis von Acryl- und Methacrylsäureglycidylestern, Polyäthylenimine und Polymere mit freien seitenständigen Aminogruppen, z.B. Poly-p-aminostyrol.

Bevorzugt sind erfindungsgemässe Polymere mit einem Durchschnittsmolekulargewicht von 1000 bis 100 000, die wiederkehrende Strukturelemente der Formel XI bis XX

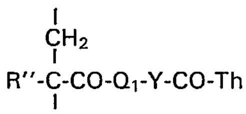
(XI)

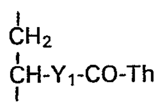
(XII)

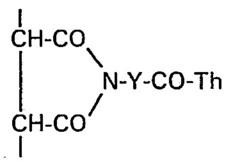
(XIII)

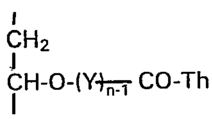
(XIV)

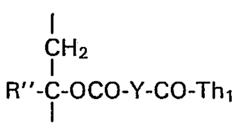
(XV)

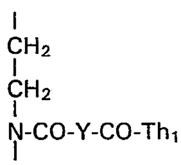
(XVI)

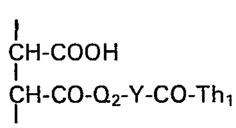
(XVII)

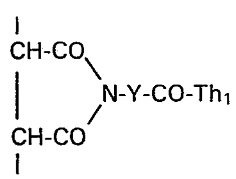
(XVIIa)

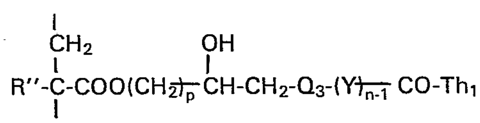
(XVIII)

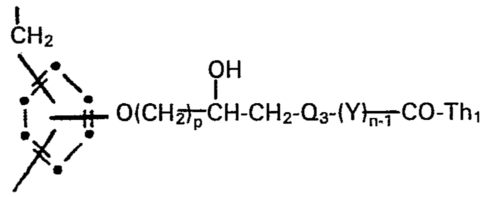
(XIX)

oder

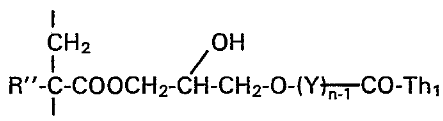
(XX)

aufweisen, worin «Th» einen Rest der Formel I''

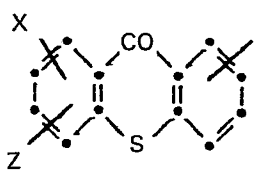
(I'')

und «$Th_1$» einen Rest der Formel I'''

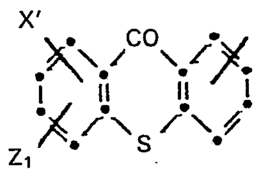
(I''')

bedeuten, X, Y, Z, R'' und n die unter Formel I und X', $Z_1$ die unter Formel If angegebene Bedeutung haben, $Q_1$ -O-, -S- oder -NH-, $Q_2$ -O-, -S-, -NH- oder -NR'-, $Q_3$ -OCO-, -O-, -S-, -NH- oder -NR'-, R' Alkyl mit 1-5 C-Atomen, p die Zahl 1 oder 2, $Y_1$ $-OR_1-$, $-SR_1-$ oder $-NHR_1-$ und $R_1$ gegebenenfalls verzweigtes Alkylen mit insgesamt 2-23 C-Atomen und 2-13 C-Atomen in der Hauptkette oder Phenylen darstellen.

Handelt es sich bei den erfindungsgemässen Polymeren um Copolymere, so bestehen diese bevorzugt aus wiederkehrenden Strukturelementen der Formeln XI bis XX und aus gleichen oder verschiedenen wiederkehrenden Strukturelementen der Formel XXI

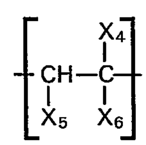
(XXI)

worin $X_5$ Wasserstoff, $X_4$ Wasserstoff, Chlor oder Methyl, $X_6$ Wasserstoff, Methyl, Chlor, CN, -COOH, $-CONH_2$, Phenyl, Methylphenyl, Methoxyphenyl, Cyclohexyl, Pyridyl, Imidazolyl, Pyrrolidyl, -COO-Alkyl mit 1-12 C-Atomen im Alkylteil, -COO-Phenyl, 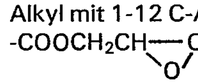, -COO-Alkyl-OH mit 1-3 C-Atomen im Alkylteil, -OCO-Alkyl mit 1-4 C-Atomen im Alkylteil, -OCO-Phenyl, -CO-Alkyl mit 1-3 C-Atomen im Alkyl, Alkoxy mit 1-20 C-Atomen oder Phenoxy oder $X_4$ Wasserstoff und $X_5$ und $X_6$ zusammen die Gruppierung -CO-O-CO- oder je -COOH oder -COO-Alkyl mit 1-6 C-Atomen im Alkyl darstellen. Bevorzugt stellt $X_5$ Wasserstoff, $X_4$ Wasserstoff

oder Methyl und $X_6$ Wasserstoff, $-OCOCH_3$, -COOH oder -COO-Alkyl mit 1-8 C-Atomen im Alkyl dar oder $X_4$ und $X_5$ bedeuten je Wasserstoff und $X_6$ stellt -CN, Chlor, Phenyl oder Alkoxy mit 1-6 C-Atomen, besonders Methoxy, dar, oder $X_4$ ist Wasserstoff und $X_5$ und $X_6$ bilden zusammen die Gruppierung -CO-O-CO-.

In den obigen Formeln I', I'', Ie, If und XI bis XX haben X bzw. X', Z bzw. $Z_1$, n, Y und $Q_4$ bzw. $Q_5$ die im Vorangehenden angegebenen entsprechenden bevorzugten Bedeutungen, und die Gruppierungen $-CO-(Y)_{n-1}-$ bzw. $-CO-(Y)_{n-1}-Q_4$ und $-CO-(Y)_{n-1}-Q_5$ sind bevorzugt in 1- oder 3-Stellung an den Benzolring gebunden. Besonders bevorzugt sind Polymere mit wiederkehrenden Strukturelementen der Formeln XI, XIII bis XV oder XVII bis XX und vor allem Polymere mit wiederkehrenden Strukturelementen der Formeln XI, XIV oder XVII und gegebenenfalls gleichen oder verschiedenen wiederkehrenden Strukturelementen der Formel XXI, worin R'' Wasserstoff oder Methyl, $Q_1$ -O-, $Q_2$ und $Q_3$ unabhängig voneinander -O- oder -NH-, p die Zahl 1, n die Zahl 1 oder 2, Y $-OR_1-$ oder $-NHR_1-$, $R_1$ Alkylen mit 2-6 C-Atomen oder, bei $Q_2$ = -O-, auch $-CH_2CH_2OCH_2-CH_2-$ oder $-(CH_2CH_2O)_2-CH_2CH_2-$, X bzw. X' Wasserstoff, Z bzw. $Z_1$ Wasserstoff oder in 7-Stellung gebundenes Methyl oder Methoxy, $X_5$ Wasserstoff, $X_4$ Wasserstoff oder Methyl und $X_6$ Wasserstoff, $-OCOCH_3$, -COOH oder -COO-Alkyl mit 1-8 C-Atomen im Alkyl oder $X_4$ und $X_5$ je Wasserstoff und $X_6$ -CN, Chlor, Phenyl oder Alkoxy mit 1-6 C-Atomen, besonders Methoxy, oder, bei $X_4$ = Wasserstoff, $X_5$ und $X_6$ zusammen auch -CO-O-CO- bedeuten und die Gruppierung $-CO-(Y)_{n-1}-$ in 1- oder 3-Stellung an den Benzolring des Restes «Th» oder «$Th_1$» gebunden sind. Besonders bevorzugte Bedeutungen von $X_4$ bis $X_6$ sind dabei $X_4$ und $X_5$ = Wasserstoff und $X_6$ = Wasserstoff, -COOH oder $C_{1-6}$-Alkoxy, besonders Methoxy, gegebenenfalls in Kombination mit Strukturelementen der Formel XXI, worin $X_4$ Wasserstoff ist und $X_5$ und $X_6$ zusammen -CO-O-CO- darstellen.

Polymere mit wiederkehrenden Strukturelementen der Formeln XI bis XIV können dadurch erhalten werden, dass man eine Verbindung der Formel Ie, worin X, Y, Z, $Q_4$ und n die oben angegebene Bedeutung haben, gegebenenfalls in Gegenwart von äthylenisch ungesättigten Comonomeren, insbesondere solchen der Formel XXIa

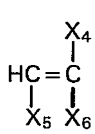 (XXIa)

worin $X_4$, $X_5$ und $X_6$ die unter Formel XXI angegebene Bedeutung haben, polymerisiert, wobei das Molverhältnis von Verbindungen der Formel Ie zu Comonomeren 1:49 bis 1:0 und insbesondere 1:4 bis 1:0 beträgt. Besonders bevorzugt verwendet man als Comonomeres Acrylsäure.

Polymere mit wiederkehrenden Strukturelementen der Formeln XV bis XX können dadurch erhalten werden, dass man

i) eine Verbindung der Formel If, worin X', $Z_1$ und Y die angegebene Bedeutung haben, $Q_5$ -COOH oder -COCl bedeutet und n die Zahl 2 ist, mit einem Polymeren mit wiederkehrenden Strukturelementen der Formeln XVa oder XVIa

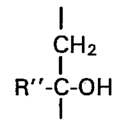 (XVa) oder 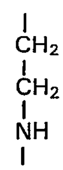 (XVIa)

ii) eine Verbindung der Formel If, worin X', $Z_1$ und Y die angegebene Bedeutung haben, $Q_5$ -OH, -SH, $-NH_2$, -NHR' oder -NCO bedeutet und n die Zahl 2 ist, mit einem Polymeren mit wiederkehrenden Strukturelementen der Formel XVIIa

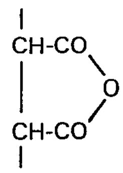 (XVIIa)

iii) eine Verbindung der Formel If, worin X', $Z_1$ und Y die angegebene Bedeutung haben und $Q_5$ -OH, -SH, $-NH_2$, -NHR' oder -COOH bedeutet und n die Zahl 2 ist, mit einem Polymeren mit wiederkehrenden Strukturelementen der Formel XVIIIa oder XIXa

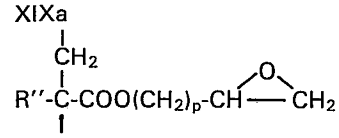 (XVIIIa)

oder

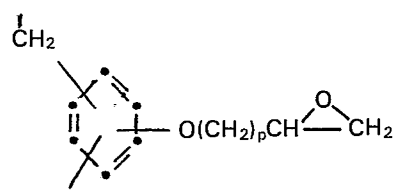

(XIXa)

worin p und R'' die unter den Formeln XVIII und XIX angegebene Bedeutung haben, oder

iv) eine Verbindung der Formel If, worin X', $Z_1$ und Y die angegebene Bedeutung haben, n die Zahl 1 oder 2 und $Q_5$ 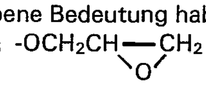 darstellt, mit einem Polymeren mit wiederkehrenden Strukturelementen der Formel XXa

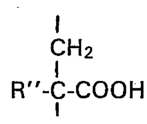 (XXa)

worin R'' die oben angegebene Bedeutung hat, zur Reaktion bringt, wobei das Verhältnis von Polymeren: Verbindung der Formel If 1:50 bis 1:1, besonders 1:5 bis 1:1, bezogen auf die Anzahl der wiederkehrenden Strukturelemente des Polymeren, beträgt. Besonders bevorzugte Polymere sind Polyacrylsäure und Maleinsäureanhydrid-Äthylen- und Maleinsäureanhydrid-Methylvinyläther-Copolymere.

Der Einbau erfindungsgemässer Thioxanthonderivate in bestehende Polymerketten mittels Kondensations- oder Additionsreaktion kann auf an sich bekannte Weise erfolgen, zweckmässig bei Temperaturen von etwa −50°C bis +150°C, und gegebenenfalls in Gegenwart eines säurebindenden Mittels, falls definitionsgemässe Salze von Verbindungen der Formel I eingesetzt werden. Die Umsetzung wird vorzugsweise in einem inerten organischen Lösungsmittel oder einem Lösungsmittelgemisch durchgeführt, für Kondensationsreaktionen bevorzugt bei Temperaturen von etwa −20°C bis +100°C. Additionsreaktionen werden zweckmässig bei erhöhter Temperatur, im allgemeinen bei Temperaturen zwischen etwa 80 und 120°C oder bei Rückflusstemperatur vorgenommen.

Geeignete Lösungsmittel zur Durchführung der Kondensations- und Additionsreaktionen sind z.B.:

- aliphatische oder cyclische Ketone, wie Aceton, Methyläthylketon, Isopropylmethylketopn, Cyclohexanon, Cyclopentanon und γ-Butyrolacton;
- cyclische Äther, wie Tetrahydrofuran, Tetrahydropyran oder Dioxan;
- cyclische Amide, wie N-Methyl-2-pyrrolidon, N-Äthyl-2-pyrrolidon oder N-Methyl-ε-caprolactam;
- N,N-Dialkylamide von aliphatischen Monocarbonsäuren mit 1-3 C-Atomen im Säureteil, wie N,N-Dimethylformamid, N,N-Dimethylacetamid, N,N-Diäthylacetamid, N,N-Dimethylmethoxyacetamid;
- Pyridin und Pyridinbasen oder tertiäre Amine, vor allem Trialkyl- und Dialkylbenzylamine mit bevorzugt 1-4 C-Atomen in den Alkylteilen, z.B. Triäthylamin und Diäthylbenzylamin;
- Dialkylsulfoxide, wie Dimethyl- und Diäthylsulfoxid.

Bevorzugte Lösungsmittel für Kondensationsreaktionen sind cyclische Amide und N,N-Dialkylamide der vorerwähnten Art, besonders N-Methyl-2-pyrrolidon, N,N-Dimethylformamid und N,N-Dimethylacetamid. Für Additionsreaktionen werden cyclische Äther und cyclische Ketone, vor allem Tetrahydrofuran und Cyclohexanon, sowie Pyridin bevorzugt.

Die Homopolymerisation von Verbindungen der Formel Ie sowie deren Copolymerisation mit anderen äthylenisch ungesättigten Monomeren, z.B. solchen der Formel XXIa, können ebenfalls auf an sich bekannte Weise vorgenommen werden, z.B. in Gegenwart üblicher kationischer und anionischer Initiatoren. Bevorzugt ist die radikalische Polymerisation. Dabei verwendet man zweckmässig etwa 0,01 bis 5 Gew.-%, vorzugsweise 0,01 bis 1,5 Gew.-%, bezogen auf das Gesamtgewicht der Monomeren, an sich bekannter Radikalinitiatoren, wie anorganische oder organische Peroxide oder Azoverbindungen, z.B. Wasserstoffperoxid, Kaliumperoxidisulfat, tert.-Butylhydroperoxid, Di-tert.-butylperoxid Peressigsäure, Benzoylperoxid, Diacylperoxide, Cumolhydroperoxid, tert.-Butylperbenzoat, tert.-Alkylperoxicarbonate und α,α′-Azoisobutyronitril. Die Reaktionstemperaturen für die radikalische Polymerisation liegen im allgemeinen zwischen etwa 30 und 100°C. Die radikalische Polymerisation kann aber auch in der Kälte durchgeführt werden, wozu man auch Redoxsysteme in den oben genannten Konzentrationen verwenden kann, beispielsweise Gemische aus Peroxiden, wie Wasserstoffperoxid, und einem Reduktionsmittel, wie zweiwertige Eisenionen.

Die Polymerisation kann in homogener Phase, z.B. in Substanz (Blockpolymerisation) oder in Lösung oder in heterogener Phase, d.h. als Fällungspolymerisation, Emulsions- oder Suspensionspolymerisation, erfolgen. Bevorzugt ist die Polymerisation in Lösung.

Geeignete Lösungsmittel sind solche der bei der Kondensations- bzw. Additionsreaktion erwähnten Art sowie:

- halogenierte aliphatische Kohlenwasserstoffe, wie Methylenchlorid, Chloroform, Tetrachloräthan und Tetrachloräthylen;
- Alkylester von aliphatischen Monocarbonsäuren mit insgesamt 2-6 C-Atomen, wie Ameisen- oder Essigsäuremethyl-, -äthyl- und -n-butylester;
- Äthylenglykoldialkyläther mit 1-4 C-Atomen in den Alkylteilen, wie Äthylenglykoldimethyl-, -diäthyl- und -di-n-butyläther.

Die erfindungsgemässen Verbindungen der Formel I können auch als solche als Sensibilisatoren für lichtvernetzbare Polymere der verschiedensten Art eingesetzt werden.

Derartige Polymere werden z.B. zur Herstellung von Druckplatten für das Offsetdruckverfahren, zur Herstellung von Photooffset-Lacken, für die unkonventionelle Photographie, z.B. zur Herstellung von photographischen Bildern mittels Photopolymerisation oder Photovernetzung verwendet. Solche Polymere finden insbesondere Anwendung als sogenannte Photoresists zur Herstellung von gedruckten Schaltungen nach an sich bekannten Methoden. Dabei wird die mit der lichtempfindlichen Schicht versehene Seite der Leiterplatte durch ein das Leiterbild aufweisendes Dianegativ belichtet und dann entwickelt, worauf man die unbelichteten Stellen der Schicht durch Entwicklungsflüssigkeit herausholt.

Als Polymere können an sich beliebige Materialien verwendet werden, deren Lichtempfindlichkeit (Empfindlichkeit gegenüber aktinischen Strahlen) sich durch den Einsatz der erfindungsgemässen Sensibilisatoren erhöhen lässt. Ganz besonders eignen sich die Verbindungen der Formel I und die daraus herstellbaren Polymeren als Sensibilisatoren für Polymere der in der deutschen Offenlegungsschrift 2 626 769 beschriebenen Art, d.h. Polymere, die als lichtempfindliche Gruppen solche der Formel XXII

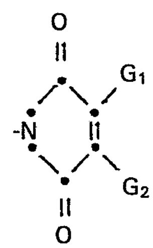

(XXII)

aufweisen, worin $G_1$ und $G_2$ unabhängig voneinander Alkyl mit 1-4 C-Atomen, besonders Methyl, oder $G_1$ und $G_2$ zusammen die Ergänzung zu einem fünf- bis sechsgliedrigen carbocyclischen Ring bedeuten.

Die Verbindungen der Formel I bzw. die daraus herstellbaren Polymeren können auf an sich bekannte Weise in die lichtvernetzbaren Polymeren eingearbeitet werden. Der Gehalt an Sensibilisator im Polymeren kann je nach Anwendungszweck und Anzahl der im Polymeren vorhandenen lichtvernetzbaren Gruppen stark variieren, liegt jedoch im allgemeinen zwischen etwa 0,1 und 20%, bezogen auf das Gewicht des Polymeren.

Schliesslich finden die erfindungsgemässen Thioxanthonderivate der Formel I sowie die daraus herstellbaren Polymeren mit seitenständigen Gruppierungen der Formel I auch Anwendung als Photoinitiatoren. Die Erfindung betrifft daher auch die Verwendung von Verbindungen der Formel I und der daraus herstellbaren Polymeren mit seitenständigen Gruppierungen der Formel I' als Initiator für die Photopolymerisation äthylenisch ungesättigter Verbindungen oder für die photochemische Vernetzung von Polyolefinen sowie Gemische aus

A) einer Verbindung der Formel I oder einem Polymeren mit seitenständigen Gruppierungen der Formel I' und

B) einem organischen Amin, als Initiatoren für die Photopolymerisation äthylenisch ungesättigter Verbindungen oder für die photochemische Vernetzung von Polyolefinen.

Die verwendeten organischen Amine können aliphatische, aromatische, araliphatische, cycloaliphatische oder heterocyclische Amine sein. Sie können primäre, sekundäre oder tertiäre Amine sein. Beispiele hierfür sind: Butylamin, Dibutylamin, Tributylamin, Cyclohexylamin, Benzyldimethylamin, Dicyclohexylamin, Triäthylamin , Phenyl-diäthanolamin, Piperidin, Piperazin, Morpholin, Pyridin, Chinolin, p-Dimethylaminobenzoesäureäthylester oder Michlers Keton (4,4'-Bis-dimethylamino-benzophenon).

Bevorzugt sind Gemische aus

A) einer Verbindung der Formel I, worin sich die Gruppe $-CO-(Y)_{n-1}$ Q in 1- oder 3-Stellung befindet und X, Z, Y, Q und n die im Vorangehenden angegebene bevorzugte Bedeutung haben, und

B) einem aliphatischen tertiären Amin, einem p-Dimethylaminobenzoesäurealkylester oder Michlers Keton.

Beispiele für aliphatische tertiäre Amine sind Trimethylamin, Triäthylamin, Tri-isopropyl-amin, Tributylamin, Dodecyl-dimethylamin, Octyldimethylamin, Triäthanolamin, Tris(hydroxypropyl)-amin, N-Methyldiäthanolamin oder N-Butyl-diäthanolamin. Besonders bevorzugt sind Gemische aus

A) einer Verbindung der Formel I, worin sich die Gruppe $-CO-(Y)_{n-1}$ Q in 1- oder 3-Stellung befindet und X, Z, Y, Q und n die im Vorangehenden angegebenen bevorzugten Bedeutungen haben, und

B) Triäthanolamin oder einem $C_{1-4}$-Alkyldiäthanolamin.

Die genannten bevorzugten Gemische enthalten die Verbindungen der Formel I und die organischen Amine bevorzugt in einem Gewichtsverhältnis von 4:1 bis 1:4.

Photopolymerisierbare Verbindungen sind beispielsweise ungesättigte Monomere wie Ester von Acryl- oder Methacrylsäure, z.B. Methyl-, Äthyl, n- oder tert.-Butyl-, Isooctyl- oder Hydroxyäthylacrylat, Methyl- oder Äthylmethacrylat, Äthylen-diacrylat, Butandioldiacrylat, Hexandioldiacrylat, Neopentyl-diacrylat, Trimethylolpropan-trisacrylat, Pentaerythrit-tetraacrylat oder Pentaerythrit-trisacrylat; Acrylnitril, Methacrylnitril, Acrylamid, Methacrylamid, N-substituierte (Meth)-acrylamide; Vinylester wie z.B. Vinyl-acetat, -propionat, -acrylat oder -succinat; sonstige Vinylverbindungen, wie Vinyläther, Vinylketone, Vinylsulfone, Styrol, Alkylstyrole, Halogenstyrole, Divinylbenzol, N,N'-Divinylharnstoff, Vinylnaphthalin, N-Vinylpyrrolidon, Vinylchlorid oder Vinylidenchlorid; Allylverbindungen, wie Diallylphthalat, Diallylmaleat, Triallylisocyanurat, Triallylphosphat oder Äthylenglykol-diallyläther und die Mischungen von solchen ungesättigten Monomeren.

Besonders geeignet sind die erfindungsgemässen Gemische für die Photopolymerisation von Acrylsäureestern und deren Gemischen.

Weitere Beispiele sind ungesättigte Acrylharze. Hierzu zählen beispielsweise Umsetzungsprodukte von Polyepoxiden (Epoxidharzen) mit Acrylsäure oder Methacrylsäure oder Umsetzungsprodukte von Polyisocyanaten mit Hydroxyalkylacrylaten sowie die Umsetzungsprodukte von hydroxylgruppenhaltigen Polyestern oder Polyäthern mit Acryl- oder Methacrylsäure. Diese ungesättigten Acrylharze werden meist im Gemisch mit einem oder mehreren Acrylaten eines Mono-, Di- oder Polyalkohols, wie z.B. Äthyl-, Butyl-, Benzyl-, 2-Äthylhexyl- oder 2-Hydroxypropylacrylat, Äthylenglykoldiacrylat, Propylenglykoldiacrylat, Butandioldiacrylat, Hexamethylen-diacrylat, Trimethylol-propan-trisacrylat oder Pentaerythrit-tetraacrylat, verwendet.

Gegenstand der Erfindung sind auch photopolymerisierbare Systeme, bestehend aus a) mindestens einer äthylenisch ungesättigten Verbindung, b) einem definitionsgemässen Gemisch aus A) und B) und gegebenenfalls c) sonstigen Zusatzstoffen, wie Inhibitoren, Stabilisatoren, UV-Absorbern, Füllstoffen, Pigmenten, Farbstoffen, Thioxotropiemittel und Verlaufshilfsmittel, z.B. Silikonöl.

Als Inhibitoren, welche vor allem während der Herstellung der Systeme durch Mischen der Komponenten vor einer vorzeitigen Polymerisation schützen sollen, werden beispielsweise Hydrochinon, Hydrochinonderivate, p-Methoxyphenol oder $\beta$-Naphthole verwendet. Als UV-Absorber können z.B. solche vom Benztriazol- oder Benzophenontyp eingesetzt werden. Als Füllstoffe kommen z.B. Kieselsäure, Talkum oder Gips in Betracht.

Bevorzugt sind derartige photopolymerisierbare Systeme in den Mengenverhältnissen 99,5-80 Gew.-% von a) und c) und 0,5-20 Gew.-% von b), wobei das Gemisch A) vorzugsweise aus einer Verbindung der Formel I besteht, worin sich die Gruppe $-CO-(Y)_{n-1}$ Q in 1- oder 3-Stellung befindet und X, Z, Y, Q und n die im Vorangehenden angegebenen bevorzugten Bedeutungen haben.

Als Komponente a) verwendet man bevorzugt einen Acrylsäureester oder ein Gemisch mehrerer Acrylsäureester.

Es können auch Kombinationen mit bekannten Photoinitiatoren, die durch Photofragmentierung Radikale bilden, wie z.B. Benzoinäther, Dialkoxyacetophenone oder Benzilketale, verwendet werden.

Grosse Bedeutung haben die erfindungsgemässen Initiatorgemische für die Photohärtung von Druckfarben und weiss pigmentierten Schichten, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Gut geeignet sind die erfindungsgemäsen Initiatoren auch für photohärtbare Systeme zur Herstellung von Druckplatten.

Ein weiteres Einsatzgebiet ist die UV-Härtung von Metallbeschichtungen, beispielsweise bei der Lackierung von Blechen für Tuben, Dosen oder Flaschenverschlüssen, sowie die UV-Härtung von Kunststoffbeschichtungen, beispielsweise von Füssböden- oder Wandbelägen auf PVC-Basis.

Beispiele für die UV-Härtung von Papierbeschichtungen sind die farblose Lackierung von Etiketten, Schallplatten-Hüllen oder Buchumschlägen.

Die erfindungsgemässen Gemische können auch als Initiatoren zur photochemischen Vernetzung von Polyolefinen verwendet werden. Hierfür kommen z.B. Polypropylen, Polybuten, Polyisobutylen sowie Copolymerisate wie z.B. Äthylen-Propylen-Copolymere in Frage, vorzugsweise jedoch Polyäthylen von niedriger, mittlerer oder hoher Dichte.

Der Zusatz der bevorzugten Photoinitiatoren zu den photopolymerisierbaren Systemen geschieht im allgemeinen durch einfaches Einführen, da die meisten dieser Systeme flüssig oder gut löslich sind. Meist kommt es zu einer Lösung der Initiatoren, wodurch deren gleichmässige Verteilung sowie die Transparenz der Polymerisate gewährleistet ist.

Die Polymerisation erfolgt nach den bekannten Methoden der Photopolymerisation durch Bestrahlung mit Licht, das reich an kurzwelliger Strahlung ist. Als Lichtquellen sind z.B. Quecksilbermitteldruck-, -hochdruck- und -niederdruckstrahler, sowie superaktinische Leuchtstoffröhren geeignet, deren Emissionsmaxima im Bereich zwischen 250 und 450 nm liegen.

Bei der photochemischen Vernetzung von Polyolefinen wird der Photoinitiator dem Polyolefin vor oder während der formgebenden Verarbeitung zugesetzt, beispielsweise durch pulverförmiges Vermischen oder durch Mischen mit dem plastifizierten Polyolefin. Die Vernetzung erfolgt durch Bestrahlung des geformten Gegenstandes in fester Form, beispielsweise in Form von Folien oder Fasern.

I) *Herstellungsbeispiele*

*Beispiel 1*

Aus 7,5 g (0,33 Grammäquivalenten) Natrium, 300 ml Methanol und 36 ml (0,33 Mol) Thiophenol hergestelltes trockenes Natriumthiophenolat wird in 300 ml Dimethylsulfoxid gelöst und mit 80,4 g (0,3 Mol) 3-Nitrophthalsäure-N-phenylimid versetzt. Das Reaktionsgemisch wird 90 Minuten auf 50°C erhitzt und dann in ein Gemisch von 300 ml Wasser und 300 ml wasserfreie Essigsäure gegossen. Die entstandene Suspension wird abgesaugt und bei 80°C/13000 Pa getrocknet. Man erhält 100 g (100% d.Th.) 3-Phenylthiophthalsäure-N-phenylimid.

99,4 g (0,3 Mol) 3-Phenylthiophthalsäure-N-phenylimid werden in 1326 ml einer 20%igen Natriumhydroxidlösung suspendiert und unter Rühren während 30 Minuten auf 100°C erhitzt. Nach dem Abkühlen wird die alkalische Suspension unter Rühren mit 672 ml 37%iger Salzsäure angesäuert. Nach einer Stunde wird die feine Suspension abgesaugt, noch nass in 882 ml 37%iger Salzsäure suspendiert und während einer Stunde zum Rückfluss erhitzt. Das Reaktionsgemisch wird abgekühlt, die entstandene feine Suspension wird abgesaugt und bei 80°C/13000 Pa getrocknet. Man erhält 69,4 g (85% d.Th.) 3-Phenylthiophthalsäure.

69 g (0,25 Mol) 3-Phenylthiophthalsäure und 700 ml Polyphosphorsäure werden unter Rühren 90 Minuten auf 200°C erhitzt, dann abgekühlt und in 3000 ml Wasser verrührt. Nach einer Stunde wird die entstandene Suspension abgesaugt, mit Wasser nachgewaschen und bei 80°C getrocknet. Das erhaltene Rohprodukt wird in 350 ml N,N-Dimethylformamid heiss gelöst, mit Tierkohle versetzt und filtriert. Das Filtrat wird mit der fünffachen Menge Wasser verdünnt, und die entstandene Suspension wird abfiltriert, mit Wasser nachgewaschen und getrocknet. Man erhält 63 g (98% d.Th.) Thioxanthon-1-carbonsäure; Fp. 259°C. Die auf diese Weise erhaltene Säure kann direkt weiterverwendet werden.

82 g (0,32 Mol) Thioxanthon-1-carbonsäure werden in 460 ml Thionylchlorid 5 Stunden unter Rückfluss gekocht. Die dabei entstehende dunkle, klare Lösung wird zur Trockne eingedampft. Man erhält 87,5 g (100% d.Th.) Thioxanthon-1-carbonsäurechlorid.

11 g (0,04 Mol) Thioxanthon-1-carbonsäurechlorid und 10,4 g (0,08 Mol) Methacrylsäure-2-hydroxyäthylester werden in 170 ml Dioxan 3 Stunden auf 80°C erhitzt und anschliessend zur Trockne eingedampft. Der feste Rückstand wird mit 100 ml Wasser verrührt, und der pH der erhaltenen Suspension wird mit einer 3%igen Natriumbicarbonatlösung auf 8 eingestellt. Dann wird das Rohprodukt mit 200 ml Methylenchlorid extrahiert, mit festem Natriumsulfat getrocknet, und das Methylenchlorid wird eingedampft. Der Rückstand wird in 100 ml Methanol gelöst und mit 4 g Tierkohle filtriert. Das auskristallisierte Produkt wird in 1200 ml Diäthyläther gelöst und mit 50 ml 1%iger Natronlauge extrahiert. Die Ätherphase wird mit Wasser gewaschen und mit Natriumsulfat getrocknet. Dann wird die ätherische Lösung mit 0,05 Gew.-% 2,6-Di-tert.-butylkresol stabilisiert und eingeengt. Man erhält 7,7 g (52% d.Th.) Thioxanthon-1-carbonsäure-$\beta$(methacryloyloxy)-äthylester; Fp. 112-113°C.

IR-Spektrum (Chloroform); 1740 $cm^{-1}$ (-COOR); 1660 $cm^{-1}$ (-CO-).

Analyse für $C_{20}H_{16}O_5S$ (Molgewicht 368,34):

| | | | |
|---|---|---|---|
| berechnet: | C 65,22 | H 4,37 | S 8,68 |
| gefunden: | C 63,8 | H 4,10 | S 8,41 |

*Beispiel 2*

18,4 g (0,06 Mol) Thioxanthon-1-carbonsäurenatriumsalz [hergestellt durch Umsetzung von Thioxanthon-1-carbonsäure mit NaOH], 14,5 g (0,18 Mol) 2-Chloräthanol und 0,18 g Diäthylamin werden

unter Rückfluss während 4 Stunden auf 130°C erhitzt. Nach dem Abkühlen der Reaktionsmasse wird diese zweimal mit je 100 ml Dioxan aufgekocht und heiss abgesaugt. Die Dioxanextrakte werden zur Trockne eingedampft. Der Rückstand wird unter Zugabe von Tierkohle aus 1000 ml Methanol umkristallisiert. Man erhält 13,2 g (73,3% d.Th.) Thioxanthon-1-carbon-säure-β-hydroxyäthylester; Fp. 169°C.

IR-Spektrum (Chloroform): 1750 $cm^{-1}$ (-COOR); 1660 $cm^{-1}$ (-CO-).

Analyse für $C_{16}H_{12}O_4S$ (Molgewicht 300,33):

| | | | |
|---|---|---|---|
| berechnet: | C 64,04 | H 4,0 | S 10,7 |
| gefunden: | C 63,8 | H 4,0 | S 10,6 |

*Beispiel 3*

90,2 g (0,726 Mol) p-Thiokresol werden in 600 ml N,N-Dimethylformamid gelöst, worauf man 31,7 g (0,792 Mol) fein pulverisiertes Natriumhydroxid zugibt. Nach halbstündiem Rühren bei 20-25°C werden zur homogenen Lösung 158,0 (0,660 Mol) Nitroterephthalsäure-dimethylester zugefügt, und das Reaktionsgemisch wird während 1,5 Stunden bei 70°C gerührt. Nach dem Abkühlen auf 20-25°C werden 1000 ml Wasser zugefügt, und der ausgefallene Niederschlag wird abfiltriert. Man gewinnt 275 g feuchtes Produkt. Dieses wird in einer Lösung von 89,5 g KOH in 1200 ml Methanol eine Stunde auf Rückfluss erhitzt. Man kühlt auf 20-25°C ab, versetzt mit 1000 ml Wasser und etwas Aktivkohle und filtriert nach halbstündigem Rühren. Das Filtrat wird am Rotationsverdampfer vom Methanol befreit, und die verbleibende wässrige Phase wird dreimal mit je 200 ml Methylenchlorid extrahiert. Der nach dem Ansäuern der wässrigen Phase mit Schwefelsäure gebildete Niederschlag wird abfiltriert und mit Wasser nachgewaschen. Nach dem Trocknen im Vakuum bei 80°C verbleiben 140 g (74% d.Th.) 2-(4-Methylphenylthio)-terephthalsäure; Fp. > 250°C.

IR-Spektrum (KBr): 1690 $cm^{-1}$.

Elementaranalyse für $C_{15}H_{12}O_4S$ (Molgewicht 288,32):

| | | |
|---|---|---|
| berechnet: | C 62,49 | H 4,20 |
| gefunden: | C 62,56 | H 4,28 |

Die 2-(4-Methylphenylthio)-terephthalsäure wird zwischen 5-10°C mit Chlorsulfonsäure behandelt, worauf man das Reaktionsgemisch auf Eis giesst und die ausgefallene Thioxanthon-7-methyl-3-carbonsäure abfiltriert und im Vakuum bei 100°C trocknet; Ausbeute 99% d.Th.

IR-Spektrum (KBr): 1640 $cm^{-1}$;

UV-Spektrum (N,N-Dimethylformamid: $\lambda_{max.}$ = 395 nm, $\epsilon$ = 5733.

80 g (0,30 Mol) der erhaltenen Thioxanthon-7-methyl-3-carbonsäure werden mit 650 ml Thionylchlorid 2 Stunden am Rückfluss gehalten, worauf man überschüssiges Thionylchlorid abdestilliert. Den Rückstand erhitzt man unter gutem Rühren mit 500 ml Äthylenglykol während 12 Stunden bei 80°C. Das Reaktionsgemisch wird bei 20-25°C mit 200 ml Wasser versetzt, und der Niederschlag wird abfiltriert. Nach dem Trocknen bei 70°C im Vakuum verbleiben 76,8 g (81% d.Th.) gelber, kristalliner Thioxanthon-7-methyl-3-carbonsäure-β-hydroxyäthylester vom Fp. 147-146°C.

IR-Spektrum (KBr): 3450 $cm^{-1}$, 1730 $cm^{-1}$, 1645 $cm^{-1}$, 1605 $cm^{-1}$.

*Beispiel 4*

Nach dem in Beispiel 3 beschriebenen Verfahren werden 5 g (0,019 Mol) Thioxanthon-7-methyl-3-carbonsäure mit 50 ml Thionylchlorid in das Säurechlorid übergeführt und dieses mit 50 ml Diäthylenglykol behandelt. Nach der Zugabe von 100 ml Wasser und Trocknen des erhaltenen Niederschlags erhält man 5,0 g (73% d.Th.) gelben Thioxanthon-7-methyl-3-carbonsäure-diäthylenglykolester; Fp. 104-109°C.

*Beispiel 5*

Nach dem in Beispiel 3 beschriebenen Verfahren erhält man aus 5 g Thioxanthon-7-methyl-3-carbonsäure, 50 ml Thionylchlorid und 60 ml Triäthylenglykol 3,1 g (42% d.Th.) wachsartigen, gelben Thioxanthon-7-methyl-3-carbonsäure-triäthylenglykolester; Schmelzbereich 38-75°C.

*Beispiel 6*

Nach dem in Beispiel 3 beschriebenen Verfahren erhält man aus 5 g Thioxanthon-7-methyl-3-carbonsäure, 60 ml Thionylchlorid und 40 ml 3-Hydroxypropionsäure 6,32 g (100% d.Th.) gelben Thioxanthon-7-methyl-3-carbonsäure-β-carboxyäthylester; Fp. > 250°C.

*Beispiel 7*

8,34 g (0,03 Mol) Thioxanthon-1-carbonsäure-Natriumsalz und 0,1 ml Triäthylamin werden in 15 ml Epichlorhydrin so lange auf 130°C erhitzt, bis sich eine dunkle homogene Lösung gebildet hat, und dann während 3 Stunden bei dieser Temperatur gehalten. Anschliessend wird das Reaktionsgemisch mit 50 ml Dioxan verdünnt, zum Rückfluss erhitzt, abgekühlt, und das ausgeschiedene Natriumchlorid wird abgesaugt. Das Lösungsmittel wird eingedampft und mit 15 ml Diäthyläther verrührt. Das auskristallisierte Rohprodukt wird abgesaugt. Sowohl das auskristallisierte Produkt als auch die Mutterlauge werden über einer Silicagel-Säule gereinigt (Laufmittel Chloroform/Aceton im Volumenverhältnis 19:1). Die nach der Chromatographie erhaltenen Produkte werden aus Methanol umkristallisiert. Man erhält 7,54 g (80,5% d.Th.) 1-Thioxanthon-1-carbonsäure-glycidylester; Fp. 115-117°C.

IR-Spektrum (Chloroform): 1750 $cm^{-1}$ (-COOR); 1660 $cm^{-1}$ (-CO-).

UV-Spektrum: $\lambda_{max.}$ = 385 nm, $\epsilon$ = 6600.

Elementaranalyse für $C_{17}H_{12}O_4S$ (Molgewicht 312,34):

| | | | |
|---|---|---|---|
| berechnet: | C 65,38 | H 3,88 | S 10,27 |
| gefunden: | C 64,62 | H 3,87 | S 10,19 |

*Beispiel 8*

0,6 g (0,01 Mol) Äthanolamin werden mit 5,6 ml äthanolischer Salzsäure (19,5%ig) vermischt. Anschliessend wird das Äthanol bis zur Trockne abdestilliert. 5,5 g (0,02 Mol) Thioxanthon-1-carbonsäurechlorid werden in 550 ml Acetonitril gelöst, und

die Lösung wird mit dem zuvor vorbereiteten Äthanolaminhydrochlorid vermischt. Das Reaktionsgemisch wird während 60 Stunden zum Rückfluss erhitzt und dann zur Trockne eingedampft. Der Rückstand wird mit 200 ml Wasser verrührt, und unlösliche Anteile werden abgesaugt. Die Lösung wird mit 10%iger wässriger Potasche alkalisch gestellt und sofort zweimal mit je 200 ml Diäthyläther ausgeschüttelt, worauf man den Diäthyläther eindampft. Man erhält 0,67 g (20% d.Th.) Thioxanthon-1-carbonsäure-$\beta$-aminoäthylester-hydrochlorid; Fp. 231 °C (Zersetzung).

IR-Spektrum (KBr): 1755 $cm^{-1}$ (-COOR); 1660 $cm^{-1}$ (-CO-).

UV-Spektrum ($H_2O$) $\lambda_{max.}$ = 390 nm, $\epsilon$ = 5000.

Elementaranalyse für $C_{16}H_{14}ClNO_3S$ (Molgewicht 335,8):

| | | | | | |
|---|---|---|---|---|---|
| ber.: | C 57,23 | H 4,20 | Cl 10,56 | N 4,17 | S 9,55 |
| gef.: | C 57,75 | H 4,5 | Cl 10,2 | N 4,2 | S 9,2 |

*Beispiel 9*

n einem 250 ml Dreihalskolben werden 19 g (0,0683 Mol) Thioxanthon-1-carbonsäure-Natriumsalz, 190 ml $\beta$-Chloräthyl-vinyläther und 0,8 g (0,0033 Mol) Triäthylmethylammoniumjodid 6 Stunden am Rückfluss gehalten. Die Reaktionslösung wird gekühlt, filtriert und dann eingedampft. Das Reaktionsprodukt wird aus Ligroin umkristallisiert. Man erhält 21,2 g (95,1% d.Th.) Thioxanthon-1-carbonsäure-$\beta$-(vinyloxy)-äthylester mit einem Schmelzpunkt von 163-164°C und einer Elementaranalyse von:

| | | | |
|---|---|---|---|
| berechnet: | C 66,24 | H 4,33 | S 9,82 |
| gefunden: | C 65,92 | H 4,21 | S 9,74 |

UV-Absorptionsspektrum: $\lambda_{max.}$ 258, 289, 302, 384 nm; $\epsilon_{max.}$ = 43400, 4700, 3900, 7000.

*Beispiel 10*

In einem 750 ml Sulfierkolben werden 20 g (0,078 Mol) Thioxanthon-1-carbonsäure und 0,1 g $Li_2(pdCl_4)$ in 400 ml Essigsäurevinylester 21 Stunden am Rückfluss gekocht. Die gräuliche Suspension wird auf 50°C gekühlt und mit 5 g Aktivkohle versetzt. Nach 15 Minuten wird die Suspension abgenutscht, und das Nutschgut wird in 1 Liter Dichlormethan 15 Minuten gekocht und heiss filtriert. Die Lösung wird eingedampft, und das erhaltene gelbe Pulver wird aus 500 ml Toluol mit Aktivkohle umkristallisiert. Man erhält 15 g (68,12% d.Th.) Thioxanthon-1-carbonsäurevinylester mit einem Schmelzpunkt von 220-222°C und einer Elementaranalyse von:

| | | | | |
|---|---|---|---|---|
| ber.: | C 68,07 | H 3,57 | O 17,0 | S 11,36 |
| gef.: | C 67,75 | H 3,56 | O 17,23 | S 11,21 |

$^1$H-NMR-Spektrum (100 MHz, $CDCl_3$ + DMSO-$d_6$): 1H : 8,5 ppm, 7H : 7,4-8,8 ppm, 2H : 4,65-5,0 ppm.

UV-Spektrum: 386 nm, $\lambda$ = 6800.

*Beispiel 11*

Zu 2,75 g (0,01 Mol) Tioxanthon-1-carbonsäurechlorid in 40 ml Dioxan werden unter Rühren während 10 Minuten 1,22 g (0,22 Mol) Äthanolamin zugetropft. Das entstandene helle Reaktionsgemisch wird 24 Stunden weitergerührt und dann zur Trockne eingeengt. Der feste Rückstand wird mit 200 ml Wasser versetzt, mit 2 × 100 ml Chloroform extrahiert, und die Extrakte werden zur Trockne eingeengt. Man erhält 2,02 g (67,5% d.Th.) Thioxanthon-1-carbonsäure-$\beta$-hydroxyäthylamid; Smp. 202°C.

IR-Spektrum (KBr): 1670 $cm^{-1}$ (-CO-NH-), 1640 $cm^{-1}$ (-CO-).

Elementaranalyse für $C_{16}H_{13}NO_3S$ (Molgewicht 299):

| | | | | |
|---|---|---|---|---|
| berechnet: | C 64,2 | H 4,4 | N 4,7 | S 10,5 |
| gefunden: | C 64,3 | H 4,4 | N 4,6 | S 10,5 |

II) *Anwendungsbeispiele*

*Beispiel I*

In einem 100 ml Dreihalskolben werden unter Stickstoff 2 g (0,007 Mol) Thioxanthon-1-carbonsäurevinylester in 49 ml N,N-Dimethylformamid gelöst. Bei 70°C gibt man 0,02 g (0,00012 Mol) Azoisobutyronitril, gelöst in 1 ml N,N-Dimethylformamid, zu und polymerisiert unter Stickstoffatmosphäre während 24 Stunden. Die Lösung wird auf 250 ml Methanol ausgefällt, und das erhaltene Polymere wird bei 40°C im Vakuum getrocknet (Polymer Nr. 1).

465,5 g (1,963 Mol) Dimethylmaleinimidyl-$\beta$--(methacryloyloxy)-äthylester [hergestellt gemäss deutscher Offenlegungsschrift 2 626 769] werden zusammen mit 49,15 g (0,49 Mol) Acrylsäureäthylester unter Stickstoff in 960 ml 1-Acetoxy-2--äthoxyäthan gelöst. Bei 80°C lässt man unter Stickstoffatmosphäre eine Lösung von 3,86 g Azoisobutyronitril in 25 ml 1-Acetoxy-2-äthoxyäthan zulaufen und polymerisiert dann während 6 Stunden. Die noch heisse Lösung wird mit 2,57 g 2,6-Di-tert.--butyl-p-kresol stabilisiert. Viskosität der Lösung, gemessen mit einem Höppler-Kugelfallviscosimeter nach DIN 53015 = 829 · $10^3$ Pa.s (Polymer Nr. 2); durchschnittliches Molekulargewicht (Streulichtmessung in Cloroform) = 1 000 000. Dieser Polymerlösung gibt man 2,7 Gew.-% des Polymeren Nr. 1) als Sensibilisator zu. Mit der auf 15 Gew.-% Feststoffgehalt verdünnten Polymerlösung werden durch Aufschleudern (500 Umdrehungen/Minute während 1 Minute) Kupferplatten so beschichtet, dass nach dem Trocknen eine 1-3 $\mu$m dicke Polymerschicht auf dem Kupfer gebildet wird. Die beschichteten Platten werden durch eine Negativvorlage (Stufenkeil Stauffer 21-Step-Sensitivity-Guide) mit einer 400 W Quecksilberhochdrucklampe in einem Abstand von 60 cm vom Vakuumtisch belichtet. Nach der Belichtung wird das Bild in einem 1,1,1-Trichloräthanbad während 2 Minuten entwickelt, wodurch die unvernetzten Anteile herausgelöst werden. Das resultierende Reliefbild des abgebildeten Stufenkeils wird durch Aetzen der blanken Kupferteile mit einer 50%igen $FeCl_3$-Lösung sichtbar gemacht. Letzte abgebildete (angeätzte) Stufe nach einer Belichtungszeit von:

| | |
|---|---|
| 1 Minute | Stufe 1 |
| 3 Minuten | Stufe 4 |
| 6 Minuten | Stufe 6 |
| 12 Minuten | Stufe 7. |

*Beispiele II*

In einem 100 ml Dreihalskolben werden 0,64 g eines handelsüblichen Copolymeren aus Aethylen und Maleinsäureanhydrid (1:1) mit einem durchschnittlichen Molekulargewicht von 20 000, 1 g (0,0035 Mol) Thioxanthon-1-carbonsäure-ß-hydroxyäthylester und 15 ml Tetrahydrofuran während 72 Stunden bei 66°C gerührt. Man erhält 1,2 g Polymer (Polymer Nr. 3).

*Beispiel III*

In einem 100 ml Dreihalskolben werden 2,5 g (0,008 Mol) Thioxanthon-1-carbonsäure-ß-(vinyloxy)äthylester, gelöst in 70 ml Toluol, vorgelegt. Über einen absteigenden Kühler werden unter Stickstoff 44 ml Toluol abdestilliert. Die erhaltene gelbe Lösung wird auf −15°C gekühlt. Bei dieser Temperatur gibt man 0.05 g frisch destilliertes Bortrifluoräthyl-ätherat zu. Die Lösung wird 3 Stunden bei −15 bis −10°C gerührt. Die gelbe Suspension wird mit 75 ml Methanol versetzt, dann abgenutscht und bei 40°C im Vakuum getrocknet. Man erhält 2,2 g (88% d.Th.) Polythioxanthon-1-carbonsäure-ß-(vinyloxy)äthylester (Polymer Nr. 4). 2,7 Gew.-% dieses Plymeren werden als Sensibilisator der in Beispiel I, Absatz 2, beschriebenen Polymerlösung zugegeben. Anschliessend werden mit der auf einen Feststoffgehalt von 15 Gew.-% verdünnten Polymerlösung Kupferplatten auf die in Beispiel I beschriebene Weise beschichtet und anschliessend belichtet.

Letzte abgebildete Stufe nach einer Belichtungszeit von:

3 Minuten Stufe 1
6 Minuten Stufe 3
12 Minuten Stufe 5

*Beispiel IV*

In einem 10 ml Dreihalskolben werden 0,744 g eines Copolymeren aus Aethylen und Maleinsäureanhydrid (1:1, mit einem durchschnittlichen Molekulargewicht von 20 000), 0,8 g (0,0024 Mol) Thioxanthon-1-carbonsäure-ß-aminoäthylester-hydrochlorid und 1,5 ml Pyridin während 24 Stunden bei 24°C gerührt. Dann wird das Reaktionsgemisch mit 5 ml Pyridin verdünnt und auf 300 ml 1N HCl ausgefällt. Die erhaltene Suspension wird abgenutscht, und das Reaktionsprodukt wird bei 40°C im Vakuum getrocknet. Man erhält 1,3 g Polymer mit einer Grenzviskosität von 0,18 dl/g in Chloroform (Polymer Nr. 5).

*Beispiel V*

7,2 g einer Polyacrylsäure mit einem durchschnittlichen Molekulargewicht von 30 000 und 21,86 g (0,07 Mol) Thioxanthon-1-carbonsäureglycidylester werden in 260 ml Cyclohexanon unter Zugabe von 0,3 Tetramethylammoniumbromid gelöst und während 6 Stunden bei 120°C unter Stickstoff gerührt. Die visköse leicht gelbe Lösung wird auf 3 Liter Diäthyläther ausgefällt. Man erhält 23,8 g (82 % d.Th.) eines Polymeren mit einer Elementaranalyse von:

ber.: C 61,56 H 4,3 O 26,42 S 7,72
gef.: C 61,43 H 4,27 O 26,43 S 7,87

Grenzviskosität des Polymeren in Chloform: 0,28 dl/g.

*Beispiel VI*

20 g (0,054 Mol) Thioxanthon-1-carbonsäure-β-(methacryloyloxy)-äthylester und 0,2 g (0,0012 Mol) Azoisobutyronitril werden in 80 ml Tetrahydrofuran unter Stickstoff gelöst und während 8 Stunden am Rückfluss gehalten. Die leicht viskose Lösung wird auf 1 Liter Diäthyläther ausgefällt, und das erhaltene weisse Polymere wird während 12 Stunden bei 40°C am Vakuum getrocknet. Man erhält 16,35 g (82% d.Th.) eines weissen Polymeren;

Grenzviskosität in Chloroform: 0,18 dl/g.

*Beispiel VII*

38,056 g (0,103 Mol) Thioxanthon-1-carbonsäure-β(methacryloyloxy)-äthylester, 7,445 g (0,103 Mol) Acrylsäure und 0,455 g (0,003 Mol) Azoisobutyronitril werden unter Stickstoff in 205 ml Tetrahydrofuran gelöst und während 8 Stunden am Rückfluss gehalten. Die farblose Lösung wird auf 2,5 Liter Diäthyläther ausgefällt, und das erhaltene Pulver wird bei 20-25°C im Vakuum getrocknet. Man erhält 34,4 g (75,6% d.Th.) eines weissen Polymeren;

Grenzviskosität in Chloroform: 0,25 dl/g.

*Beispiel VIII*

12,88 g Thioxanthon-7-methyl-3-carbonsäure-diäthylenglykolester und 5 g eines Copolymeren aus Methylvinyläther und Maleinsäureanhydrid (1:1) werden unter Zugabe von 1 ml Pyridin in 180 ml Tetrahydrofuran gelöst und während 48 Stunden bei 70°C gerührt. Die gelbe Polymerlösung wird auf 2 Liter Diäthyläther ausgefällt, und das erhaltene Polymerpulver wird bei 40°C im Vakuum getrocknet. Man erhält 16,5 g (92,3% d.Th.) Polymer;

Grenzviskosität in Chloroform: 0,30 dl/g.

*Beispiel IX*

Es wird ein Weisslack nach folgender Rezeptur hergestellt:

1,89 g Plex 6631 (Acrylharz der Fa. Röhm + Haas, Bundesrepublik Deutschland)
0,52 g 2-Hydroxypropylacrylat
2,40 g Titandioxid RTC-2 (Titandioxid der Fa. BTP Thioxide, England)
0,13 g N-Methyldiäthanolamin
1,29 g Hexandioldiacrylat
0,03 g Thioxanthon-1-carbonsäure-β-hydroxyäthylester.

Das Gemisch wird mit Hilfe einer Tellerreibmaschine mit einem Auflagegewicht von 7,5 kg vermahlen (200 Runden). Der so hergestellte Weisslack wird mit einem 40 μm Rakel auf Glasplatten aufgetragen. Die Proben werden mit einem UV-Belichtungsgerät (Standard-Hg-Dampflampe; Lampenleistung 80 W/cm, Lampenabstand 11 cm, Transportbandgeschwindigkeit = 50 m/Min.) bestrahlt. Zur Beurteilung der Aushärtung der Weisslackproben werden die folgenden vier Test herangezogen:

1. *Wischfestigkeit: 4.*

Dabei wird die Anzahl der Durchgänge der Probe

durch das Bestrahlungsgerät bis zum Erhalten einer wischfesten Oberfläche festgestellt.

2. *Pendelhärte: 142.*
Die Proben werden 10 mal durch das UV-Bestrahlungsgerät geführt. Danach wird die Pendelhärte gemäss DIN 53157 bestimmt.

3. *Glanz: 81.*
Die Proben werden 10 mal durch das UV-Bestrahlungsgerät geführt. Die Glanzmessung erfolgt mit Hilfe eines Multigloss-Geräts bei einem Winkel von 60° (DIN 67 530).

4. *Weisston: 0.*
Die Proben werden 10 mal durch das UV-Bestrahlungsgerät geführt. Der Weisston (Yellowness Index) wird mit Hilfe eines Farbmessgerätes bestimmt.

**Patentansprüche**

1. Verbindungen der Formel I

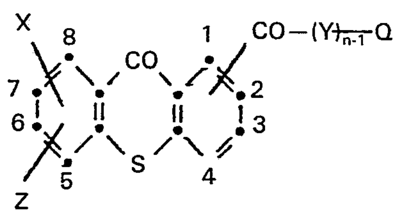

worin n die Zahl 1 oder 2, X Wasserstoff, Halogen, -CN, -OH, -SH, $-NO_2$, $-NH_2$, Phenylsulfonyl, Alkylsulfonyl, Alkyl, Alkoxy, Alkylthio, N,N-Dialkylamino oder -CO-Alkyl mit je 1-4 C-Atomen in den Alkylteilen, Z Wasserstoff, Halogen, -OH, -SH, Alkyl, Alkoxy, Alkylthio oder N,N-Dialkylamino mit je 1-4 C-Atomen in den Alkylteilen, Y $-OR_1-$, $-SR_1-$ oder $-N(R_2)R_1-$, $R_1$ gegebenenfalls verzweigtes Alkylen mit insgesamt 2-23 C-Atomen und 2-13 C-Atomen in der Hauptkette, Cyclopentylen, Cyclohexylen, Phenylen,

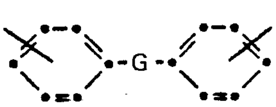,

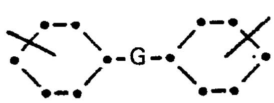

oder, wenn n = 2 und Q -OH oder $-OCO-C(R'')=CH_2$ sind, auch $-(CH_2CH_2O)_x-CH_2CH_2-$, $R_2$ Wasserstoff oder gegebenenfalls verzweigtes Alkyl mit insgesamt 1-23 C-Atomen und 1-13 C-Atomen in der Hauptkette, G $-CH_2-$, $-CH_2CH_2-$, $-C(CH_3)_2-$, -O-, -SO- oder -NH-, x eine ganze Zahl von 1-5, Q, wenn n = 1 ist, $-OCH_2CH-CH_2$ (O), $-OCH_2-COOH$, $-OCH=CH_2$, $-OCH_2CH=CH_2$, $-SCH_2CH=CH_2$ oder $-NHCH_2CH=CH_2$ und wenn n = 2 ist, -OH, -SH, $-NH_2$, -NHR', $-SO_3H$, -COOH, -COCl, -NCO, $-OCO-C(R'')=CH_2$, $-SCO-C(R'')=CH_2$, $-NHCO-C(R'')=CH_2$, $-OCH=CH_2$, $-OCH_2CH-CH_2$ (O), -N(CO)(CO) oder, wenn $R_1$ Alkylen oder Phenylen bedeutet, auch $-CH=CH_2$, R' Alkyl mit 1-5 C-Atomen und R'' Wasserstoff oder Methyl bedeuten, sowie Salze von Verbindungen der Formel I, worin Q $-NH_2$ oder -NHR' darstellt.

2. Verbindungen der Formel I nach Anspruch 1, worin Z Wasserstoff bedeutet, n, X, Y und Q die angegebene Bedeutung haben und sich die Gruppierung $-CO-(Y)_{n-1}Q$ in 1- oder 3-Stellung befindet.

3. Verbindungen der Formel I nach Anspruch 1, worin X Wasserstoff und Z Wasserstoff oder in 7-Stellung gebundenes Methyl oder Methoxy bedeuten, die Gruppe $-CO-(Y)_{n-1}Q$ sich in 1- oder 3-Stellung befindet, n = 1 ist und Q $-OCH_2CH-CH_2$ (O) oder $-OCH=CH_2$ darstellt oder n = 2 ist, Y $-OR_1-$ oder $-NHR_1-$, $R_1$ Alkylen mit 2-6 C-Atomen oder bei Q = -OH oder $-OCO-C(R'')=CH_2$ auch $-CH_2CH_2-OCH_2CH_2-$ oder $-(CH_2CH_2O)_2CH_2CH_2-$ und Q -OH, $-NH_2$, $-NHCH_3$, -COOH, -COCl, $-OCO-C(R'')=CH_2$, $-OCH=CH_2$, $-OCH_2CH-CH_2$ (O) oder -N(CO)(CO) darstellen, sowie Salze von derartigen Verbindungen der Formel I, worin Q $-NH_2$ oder $-NHCH_3$ ist, mit anorganischen Säuren.

4. Verbindungen der Formel I nach Anspruch 3, worin Q bei n = 1 $-OCH=CH_2$ und bei n = 2 -OH, $-NH_2$, $-OCH=CH_2$ oder $-OCO-C(R'')=CH_2$ darstellt, sowie Salze von derartigen Verbindungen der Formel I, worin Q $-NH_2$ ist, mit HCl.

5. Ein Verfahren zur Herstellung von Verbindungen der Formel I bzw. definitionsgemässen Salzen davon, nach Anspruch 1, dadurch gekennzeichnet, dass man

a) eine Verbindung der Formel II

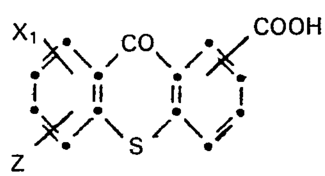

(II)

einen $C_{1-6}$-Alkylester einer Verbindung der Formel II oder ein Säurechlorid einer Verbindung der Formel II mit Z und $X_1$ ungleich -SH oder -OH mit einer Verbindung der Formel IIIa

$$H(Y)_{n-1}Q' \quad \text{(IIIa)}$$

oder, bei Q' = $-NH_2$ oder -NHR', einem Salz einer Verbindung der Formel IIIa zu einer Verbindung der Formel Ia

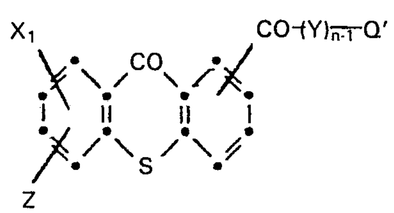

(Ia)

bzw. entsprechenden Salzen umsetzt,

b) eine Verbindung der Formel II oder einen $C_{1-6}$-Alkylester einer Verbindung der Formel II mit einer Verbindung der Formel IIIb

$$CH_2=CH-OCO-R' \quad \text{(IIIb)}$$

zu einer Verbindung der Formel Ib

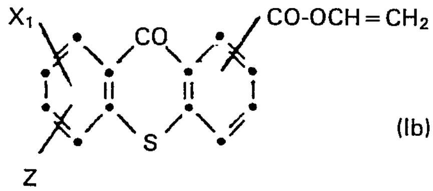

(Ib)

umsetzt, oder

c) ein Säurechlorid einer Verbindung der Formel II zuerst mit einem Salz einer Verbindung der Formel IIIc

$H-Y-NH_2$ (IIIc)

zum entsprechenden Salz einer Verbindung der Formel IV

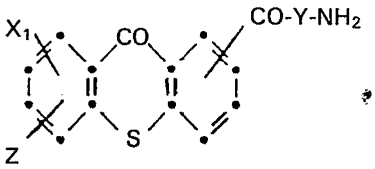

(IV)

umsetzt und das Salz einer Verbindung der Formel IV in Gegenwart eines inerten organischen Lösungsmittels mit Phosgen zu einer Verbindung der Formel Ic

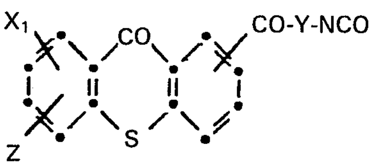

(Ic)

umsetzt, wobei Y, Z, R' und n die unter Formel I angegebene Bedeutung haben, $X_1$ Wasserstoff, Halogen, -CN, -OH, -SH, $-NO_2$, Phenylsulfonyl, Alkylsulfonyl, Alkyl, Alkoxy, Alkylthio, N,N-Dialkylamino oder -CO-Alkyl mit je 1-4 C-Atomen in den Alkylteilen darstellt, Q', bei n = 1, 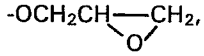, $-OCH_2-COOH$, $-OCH_2CH=CH_2$, $-SCH_2CH=CH_2$ oder $-NHCH_2CH=CH_2$ und bei n = 2, -OH, -SH, $-NH_2$, -NHR', $-SO_3H$, -COOH, -OCO-C(R'')=$CH_2$, -SCO-C(R'')=$CH_2$, -NHCO-C(R'')=$CH_2$, 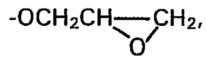, 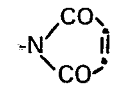 oder, wenn $R_1$ Alkylen oder Phenylen bedeutet, auch $-CH=CH_2$ und R'' Wasserstoff oder Methyl darstellen, und gegebenenfalls anschliessend die Gruppe $X_1$ in den Verbindungen der Formeln Ia, Ib oder Ic in eine davon verschiedene Gruppe X und/oder die Gruppe Q' = -COOH in Formel Ia durch Behandlung mit geeigneten Chlorierungsmitteln in die Gruppe -COCl überführt.

6. Ein Polyymeres, dessen Durchschnittsmolekulargewicht mindestens 1000 beträgt und das seitenständige Thioxanthongruppierungen der Formel I'

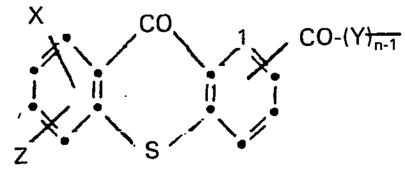

(I')

aufweist, worin n die Zahl 1 oder 2, X Wasserstoff, Halogen, -CN, -OH, -SH, $-NH_2$, $-NO_2$, Phenylsulfonyl, Alkylsulfonyl, Alkyl, Alkoxy, Alkylthio, N,N-Dialkylamino oder -CO-Alkyl mit je 1-4 C-Atomen in den Alkylteilen, Z Wasserstoff, Halogen, -OH, -SH, Alkyl, Alkoxy, Alkylthio oder N,N-Dialkylamino mit je 1-4 C-Atomen in den Alkylteilen, Y $-OR_1$, $-SR_1$- oder $-N(R_2)R_1$-, $R_1$ gegebenenfalls verzweigtes Alkylen mit insgesamt 2-23 C-Atomen und 2-13 C-Atomen in der Hauptkette, Cyclopentylen, Cyclohexylen, Phenylen,

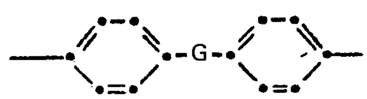 , 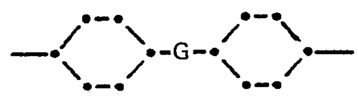

oder $-(CH_2CH_2O)_x-CH_2CH_2-$, $R_2$ Wasserstoff oder gegebenenfalls verzweigtes Alkyl mit insgesamt 1-23 C-Atomen und 1-13 C-Atomen in der Hauptkette, G $-CH_2-$, $-CH_2CH_2-$, $-C(CH_3)_2-$, -O-, $-SO_2-$ oder -NH- und x eine ganze Zahl von 1-5 bedeuten, wobei der Anteil an Gruppierungen der Formel I' mindestens 2 Prozent, bezogen auf die Anzahl der wiederkehrenden Strukturelemente des Polymeren, beträgt.

7. Ein Polymers nach Anspruch 6, worin der Anteil an Gruppierungen der Formel I' 20-100 Prozent, bezogen auf die Anzahl der wiederkehrenden Strukturelemente des Polymeren, beträgt.

8. Ein Polymeres nach Anspruch 6, mit einem Durchschnittsmolekulargewicht von 1000 bis 100000, das wiederkehrende Strukturelemente der Formel XI bis XX

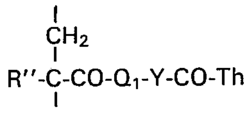

(XI)

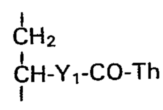

(XII)

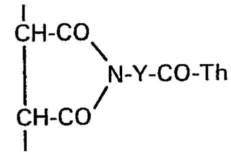

(XIII)

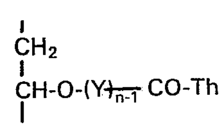

(XIV)

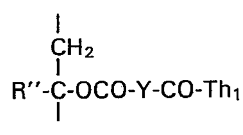

(XV)

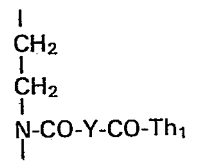

(XVI)

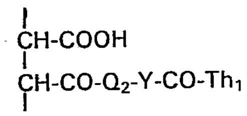

(XVII)

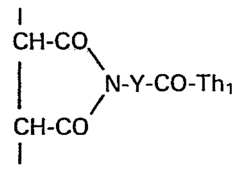

(XVIIa)

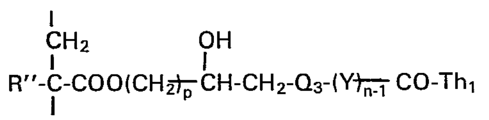

(XVIII)

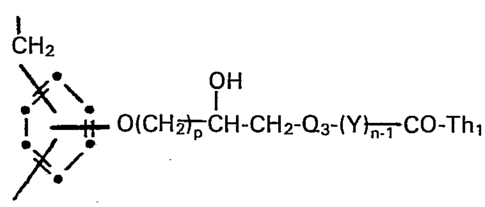

(XIX)

oder

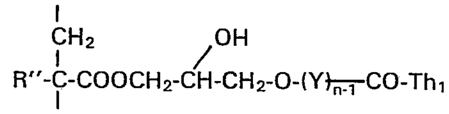

(XX)

aufweist, worin «Th» einen Rest der Formel I''

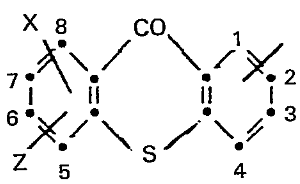

(I'')

und «$Th_1$» einen Rest der Formel I'''

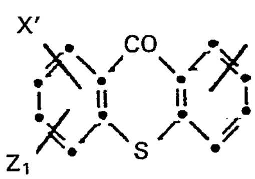

(I''')

bedeuten, X, Y, Z, und n die unter Formel I' angegebene Bedeutung haben, X' Wasserstoff, Halogen, -CN, $-NO_2$, Phenylsulfonyl, Alkylsulfonyl, Alkyl, Alkoxy, Alkylthio, N,N-Dialkylamino oder -CO-Alkyl mit je 1-4 C-Atomen in den Alkylteilen, $Z_1$ Wasserstoff, Halogen, Alkyl, Alkoxy, Alkylthio oder N,N-Dialkylamino mit je 1-4 C-Atomen in den Alkylteilen, $Q_1$ -O-, -S- oder -NH-, $Q_2$ -O-, -S-, -NH- oder -NR'-, $Q_3$ -OCO-, -O-, -S-, -NH- oder -NR'-, R' Alkyl mit 1-5 C-Atomen, R'' Wasserstoff oder Methyl, p die Zahl 1 oder 2, $Y_1$ $-OR_1-$, $-SR_1-$ oder $-NH-R_1-$ und $R_1$ gegebenenfalls verzweigtes Alkylen mit insgesamt 2-23 C-Atomen und 2-13 C-Atomen in der Hauptkette oder Phenylen bedeuten.

9. Ein Copolymeres nach Anspruch 8, das aus wiederkehrenden Strukturelementen der Formeln XI bis XX und aus gleichen oder verschiedenen wiederkehrenden Strukturelementen der Formel XXI

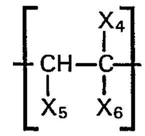

(XXI) besteht,

worin $X_5$ Wasserstoff, $X_4$ Wasserstoff, Chlor oder Methyl, $X_6$ Wasserstoff, Methyl, Chlor, CN, -COOH, $-CONH_2$, Phenyl, Methylphenyl, Methoxyphenyl, Cyclohexyl, Pyridyl, Imidazolyl, Pyrrolidyl, -COO-Alkyl mit 1-1.2 C-Atomen im Alkylteil, -COO-Phenyl, 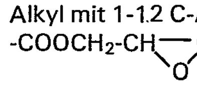, -COO-Alkyl-OH mit 1-3 C-Atomen im Alkylteil, -OCO-Alkyl mit 1-4 C-Atomen im Alkylteil, -OCO-Phenyl, -CO-Alkyl mit 1-3 C-Atomen im Alkyl, Alkoxy mit 1-20 C-Atomen oder Phenoxy oder $X_4$ Wasserstoff und $X_5$ und $X_6$ zusammen die Gruppierung -CO-O-CO- oder je -COOH oder -COO-Alkyl mit 1-6 C-Atomen im Alkyl darstellen.

10. Ein Polymeres nach Anspruch 8 oder 9, das wiederkehrende Strukturelemente der Formeln XI, XIII bis XV oder XVII bis XX und insbesondere wiederkehrende Strukturelemente der Formeln XI, XIV oder XVII, sowie gegebenenfalls gleiche oder verschiedene wiederkehrende Strukturelemente der Formel XXI aufweist, worin R'' Wasserstoff oder Methyl, $Q_1$ -O-, $Q_2$ und $Q_3$ unabhängig voneinander -O- oder -NH-, p die Zahl 1, n die Zahl 1 oder 2, Y $-OR_1-$ oder $-NHR_1-$, $R_1$ Alkylen mit 2-6 C-Atomen oder bei $Q_2$ = -O- auch $-CH_2CH_2OCH_2CH_2-$ oder $-(CH_2CH_2O)_2-CH_2CH_2-$, X bzw. X' Wasserstoff, Z bzw. $Z_1$ Wasserstoff oder in 7-Stellung gebundenes Methyl oder Methoxy, $X_5$ Wasserstoff, $X_4$ Wasserstoff oder Methyl und $X_6$ Wasserstoff, $-OCOCH_3$, -COOH oder -COO-Alkyl mit 1-8 C-Atomen im Alkyl oder $X_4$ und $X_5$ je Wasserstoff und $X_6$ -CN, Chlor, Phenyl oder Alkoxy mit 1-6 C-Atomen oder, bei $X_4$ = Wasserstoff, $X_5$ und $X_6$ zusammen auch -CO-O-CO- bedeuten und die Gruppierungen $-CO-(Y)_{n-1}-$ in 1- oder 3-Stellung an dem Benzolring des Restes «Th» oder «$Th_1$» gebunden sind.

11. Ein Verfahren zur Herstellung eines Polymeren nach Anspruch 6, dadurch gekennzeichnet, dass man entweder

A) eine Verbindung der Formel Ie

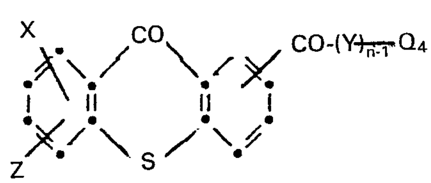

(Ie)

worin $Q_4$ bei n = 1, $-OCH{=}CH_2$, $-OCH_2CH{=}CH_2$, $-SCH_2CH{=}CH_2$ oder $-NHCH_2CH{=}CH_2$, und bei n =

2 $-OCO-C(R'')=CH_2$, $-SCO-C(R'')=CH_2$, $-NHCO-C(R'')=CH_2$, $-OCH=CH_2$, 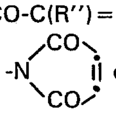 oder, wenn $R_1$ Alkylen oder Phenylen bedeutet, auch $-CH=CH_2$ darstellen, X, Y, Z und n die unter Formel I' angegebene Bedeutung haben, gegebenenfalls in Gegenwart von Comonomeren zur Reaktion bringt, wobei das Molverhältnis von Verbindung der Formel Ie zu Comonomeren 1:49 bis 1:0 beträgt, oder

B) eine Verbindung der Formel If

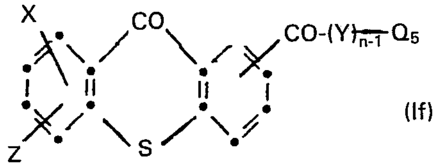

(If)

worin X' Wasserstoff, Halogen, -CN, $-NO_2$, Phenylsulfonyl, Alkylsulfonyl, Alkyl, Alkoxy, Alkylthio, N,N-Dialkylamino oder -CO-Alkyl mit je 1-4 C-Atomen in den Alkylteilen, $Z_1$ Wasserstoff, Halogen, Alkyl, Alkoxy, Alkylthio oder N,N-Dialkylamino mit je 1-4 C-Atomen in den Alkylteilen und $Q_5$ bei n = 1, $-OCH_2\underset{O}{\underbrace{CH-CH_2}}$ oder $-OCH_2-COOH$ und bei n = 2 -OH, -SH, $-NH_2$, -NHR', $-SO_3H$, -COOH, -COCl, -NCO oder $-OCH_2\underset{O}{\underbrace{CH-CH_2}}$ darstellen und Y und n die unter Formel I' angegebene Bedeutung haben, und R' Alkyl mit 1-5 C-Atomen darstellt, oder Salze von Verbindungen der Formel If, worin $Q_5$-$NH_2$ oder -NHR' darstellt, mit einem Polymeren mit entsprechenden funktionellen Gruppen in einem Verhältnis von 1:50 bis 1:1, bezogen auf die Anzahl der wiederkehrenden Strukturelemente des Polymeren, zur Reaktion bringt.

12. Verwendung einer Verbindung der Formel I nach Anspruch 1 oder eines Polymeren nach Anspruch 6 als Sensibilisator für lichtvernetzbare Polymere.

13. Verwendung einer Verbindung der Formel I nach Anspruch 1 oder eines Polymeren nach Anspruch 6 als Initiator für die Photopolymerisation äthylenisch ungesättigter Verbindungen oder für die photochemische Vernetzung von Polyolefinen.

## Claims

1. A compound of the formula I

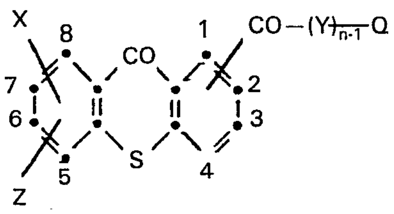

in which n is the number 1 or 2, X is hydrogen, halogen, -CN, -OH, -SH, $-NO_2$, $-NH_2$, phenylsulfonyl or alkylsulfonyl, alkyl, alkoxy, alkylthio, N,N-dialkylamino or -CO-alkyl having, in each case, 1-4 C atoms in the alkyl moieties, Z is hydrogen, halogen, -OH, -SH or alkyl, alkoxy, alkylthio or N,N-dialkylamino having, in each case, 1-4 C atoms in the alkyl moieties, Y is $-OR_1-$, $-SR_1-$ or $-N(R_2)R_1-$, $R_1$ is straight-chain or branched alkylene having a total of 2-23 C atoms and 2-13 C atoms in the main chain, cyclopentylene, cyclohexylene, phenylene,

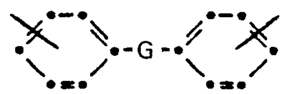 , 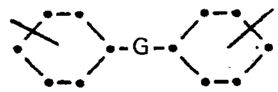

or, if n is 2 and Q is -OH or $-OCO-C(R'')=CH_2$, also $-(CH_2CH_2O)_x-CH_2CH_2-$, $R_2$ is hydrogen or straight-chain or branched alkyl having a total of 1-23 C atoms and 1-13 C atoms in the main chain, G is $-CH_2-$, $-CH_2CH_2-$, $-C(CH_3)_2-$, -O-, $-SO_2-$ or -NH-, x is an integer from 1 to 5, Q is $-OCH_2\underset{O}{\underbrace{CH-CH_2}}$, $-OCH_2-COOH$, $-OCH=CH_2$, $-OCH_2CH=CH_2$, $-SCH_2CH=CH_2$ or $-NHCH_2CH=CH_2$ if n is 1, and is -OH, -SH, $-NH_2$, -NHR', $-SO_3H$, -COOH, -COCl, -NCO, $-OCO-C(R'')=CH_2$, $-SCO-C(R'')=CH_2$, $-NHCO-C(R'')=CH_2$, $-OCH=CH_2$, $-OCH_2\underset{O}{\underbrace{CH-CH_2}}$ or 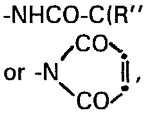, if n is 2, or is also $-CH=CH_2$ if $R_1$ is alkylene or phenylene, and R' is alkyl having 1-5 C atoms and R'' is hydrogen or methyl, or a salt of a compound of the formula I in which Q is $-NH_2$ or -NHR'.

2. A compound of the formula I according to claim 1, in which Z is hydrogen, n, X, Y and Q are as defined and the grouping $-CO-(Y)_{n-1}$ Q is in the 1-position or 3-position.

3. A compound of the formula I according to claim 1, in which X is hydrogen and Z is hydrogen or methyl or methoxy bonded in the 7-position, the group $-CO-(Y)_{n-1}$ Q is in the 1- or 3-position, n is 1 and Q is $-OCH_2\underset{O}{\underbrace{CH-CH_2}}$ or $-OCH=CH_2$, or n is 2, Y is $-OR_1-$ or $-NHR_1-$, $R_1$ is alkylene having 2-6 C atoms or, if Q is -OH or $-OCO-C(R'')=CH_2$, also $-CH_2CH_2-OCH_2-CH_2-$ or $-(CH_2CH_2O)_2CH_2CH_2-$ and Q is -OH, $-NH_2$, $-NHCH_3$, -COOH, -COCl, $-OCO-C(R'')=CH_2$, $-OCH=CH_2$, $-OCH_2\underset{O}{\underbrace{CH-CH_2}}$ or 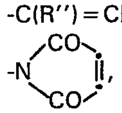, or a salt of such a compound of the formula I, in which Q is $-NH_2$ or $-NHCH_3$, with an inorganic acid.

4. A compound of the formula I according to claim 3, in which Q is $-OCH=CH_2$ if n is 1, and is -OH, $-NH_2$, $-OCH=CH_2$ or $-OCO-C(R'')=CH_2$ if n is 2, or a salt of such a compound of the formula I, in which Q is $-NH_2$, with HCl.

5. A process for the preparation of a compound of the formula I or a salt thereof according to the definition, according to claim 1, which comprises

a) reacting a compound of the formula II

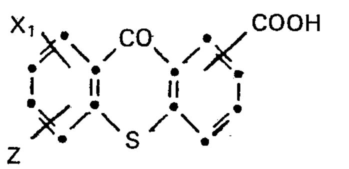

(II)

a $C_{1-6}$-alkyl ester of a compound of the formula II or an acid chloride of a compound of the formula II, in which Z and $X_1$ are not -SH or -OH, with a compound of the formula IIIa

$$H(Y)_{\overline{n-1}}Q' \qquad (IIIa)$$

or, if Q' is $-NH_2$ or -NHR', with a salt of a compound of the formula IIIa, to give a compound of the formula Ia

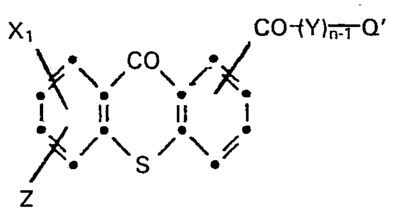

(Ia)

or corresponding salts,

b) reacting a compound of the formula II or a $C_{1-6}$-alkyl ester of a compound of the formula II with a compound of the formula IIIb

$$CH_2{=}CH\text{-}OCO\text{-}R' \qquad (IIIb)$$

to give a compound of the formula Ib

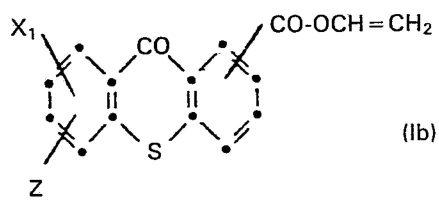

(Ib)

or

c) first reacting an acid chloride of a compound of the formula II with a salt of a compound of the formula IIIc

$$H\text{-}Y\text{-}NH_2 \qquad (IIIc)$$

to give the corresponding salt of a compound of the formula IV

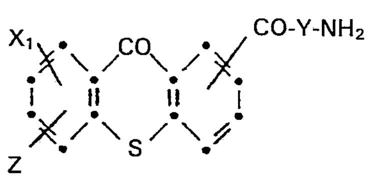

(IV)

and reacting the salt of a compound of the formula IV, in the presence of an inert organic solvent, with phosgene to give a compound of the formula Ic

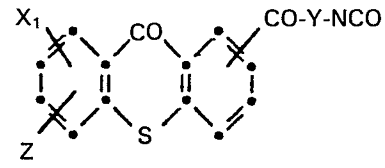

(Ic)

in which formulae Y, Z, R' and n are as defined under formula I, $X_1$ is hydrogen, halogen, -CN, -OH, -SH, $-NO_2$, phenylsulfonyl or alkylsulfonyl, alkyl, alkoxy, alkylthio, N,N-dialkylamino or -CO-alkyl having, in each case, 1-4 C atoms in the alkyl moieties, Q' is $-OCH_2CH\underset{O}{—}CH_2$, $-OCH_2\text{-}COOH$, $-OCH_2CH{=}CH_2$, $-SCH_2CH{=}CH_2$ or $-NHCH_2CH{=}CH_2$ if n is 1, and is -OH, -SH, $-NH_2$, -NHR', $-SO_3H$, -COOH, $-OCO\text{-}C(R''){=}CH_2$, $-SCO\text{-}C(R''){=}CH_2$, $-NHCO\text{-}C(R''){=}CH_2$, $-OCH_2CH\underset{O}{—}CH_2$ or -N 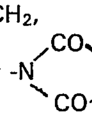 if n is 2, or is also $-CH{=}CH_2$ if $R_1$ is alkylene or phenylene, and R'' is hydrogen or methyl, and, if desired, subsequently converting the group $X_1$ in the compounds of the formulae Ia, Ib or Ic into a group X which differs from $X_1$, and/or converting the group Q' = -COOH in formula Ia into the group -COCl by treatment with suitable chlorinating agents.

6. A polymer which has a mean molecular weight of not less than 1000 and which contains, in side chains, thioxanthone groupings of the formula I'

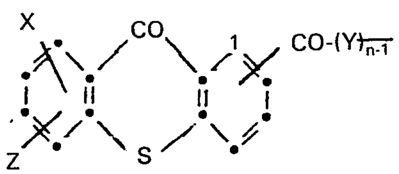

(I')

in which n is the number 1 or 2, X is hydrogen, halogen, -CN, -OH, -SH, $-NH_2$, $-NO_2$, phenylsulfonyl or alkylsulfonyl, alkyl, alkoxy, alkylthio, N,N-dialkylamino or -CO-alkyl having, in each case, 1-4 C atoms in the alkyl moieties, Z is hydrogen, halogen, -OH, -SH or alkyl, alkoxy, alkylthio or N,N-dialkylamino having, in each case, 1-4 C atoms in the alkyl moieties, Y is $-OR_1$, $-SR_1$- or $-N(R_2)R_1$-, $R_1$ is straight-chain or branched alkylene having a total of 2-23 C atoms and 2-13 C atoms in the main chain, cyclopentylene, cyclohexylene, phenylene,

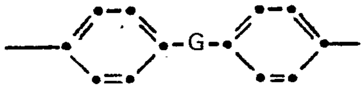 , 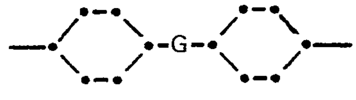

or $-(CH_2CH_2O)_x\text{-}CH_2CH_2$-, $R_2$ is hydrogen or straight-chain or branched alkyl having a total of 1-23 C atoms and 1-13 C atoms in the main chain, G is $-CH_2$-, $-CH_2CH_2$-, $-C(CH_3)_2$-, -O-, $-SO_2$- or -NH- and x is an integer from 1 to 5, the proportion of groupings of the formula I' being not less than 2 per cent, based on the number of recurring structural elements of the polymer.

7. A polymer according to claim 6, in which the proportion of groupings of the formula I' is 20-100 per cent, based on the number of recurring structural elements in the polymer.

8. A polymer according to claim 6, which has a mean molecular weight of about 1,000 to 100,000 and contains recurring structural elements of the formulae XI to XX

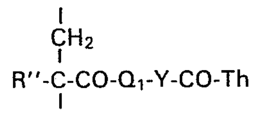

(XI)

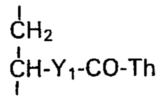

(XII)

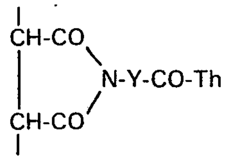

(XIII)

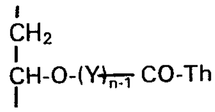

(XIV)

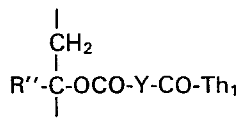

(XV)

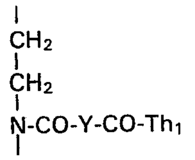

(XVI)

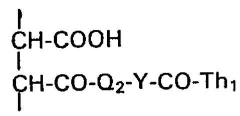

(XVII)

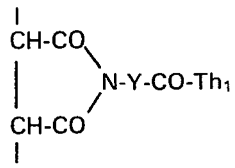

(XVIIa)

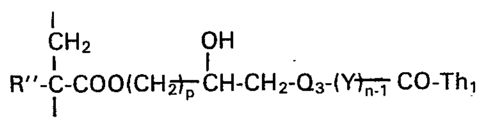

(XVIII)

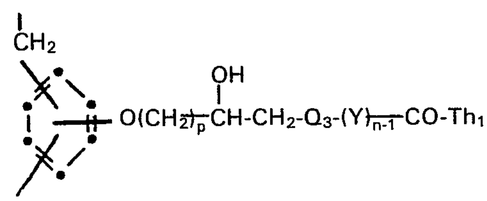

(XIX)

or

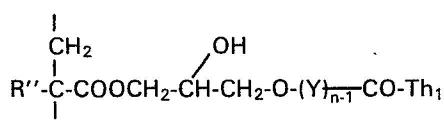

(XX)

in which «Th» is a radical of the formula I''

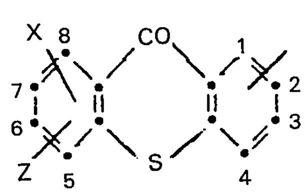

(I'')

and «$Th_1$» is a radical of the formula III'''

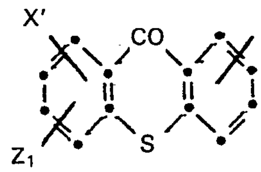

(I''')

X, Y, Z and n are as defined under formula I', X' is hydrogen, halogen, -CN, $-NO_2$, phenylsulfonyl or alkylsulfonyl, alkyl, alkoxy, alkylthio, N,N-dialkylamino or -CO-alkyl having, in each case, 1-4 C atoms in the alkyl moieties, $Z_1$ is hydrogen, halogen or alkyl, alkoxy, alkylthio or N,N-dialkylamino having, in each case, 1-4 C atoms in the alkyl moieties, $Q_1$ -O-, -S- or -NH-, $Q_2$ -O-, -S-, -NH- or -NR'-, $Q_3$ is -OCO-, -O-, -S-, -NH- or -NR'-, R' is alkyl having 1-5 C atoms, R'' is hydrogen or methyl, p is the number 1 or 2, $Y_1$ is $-OR_1-$, $-SR_1-$ or $-NH-R_1-$ and $R_1$ is straight-chain or branched alkylene having a total of 2-23 C atoms and 2-13 C atoms in the chain, or phenylene.

9. A polymer according to claim 8, which consists of recurring structural elements of the formulae XI to XX and of identical or different recurring structural elements of the formula XXI

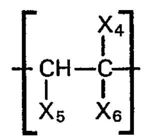

(XXI)

in which $X_5$ is hydrogen, $X_4$ is hydrogen, chlorine or methyl and $X_6$ is hydrogen, methyl, chlorine, CN, -COOH, $-CONH_2$, phenyl, methylphenyl, methoxyphenyl, cyclohexyl, pyridyl, imidazolyl, pyrrolidyl, -COO-alkyl having 1-12 C atoms in the alkyl moiety, -COO-Phenyl, $-COOCH_2-$ 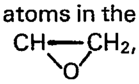, -COO-alkyl-OH having mit 1-3 C atoms in the alkyl moiety, -OCO-alkyl having 1-4 C atoms in the alkyl moiety, -OCO-phenyl, -CO-alkyl having 1-3 C atoms in the alkyl, alkoxy having 1-20 C atoms or phenoxy, or $X_4$ is hydrogen and $X_5$ and $X_6$ together are the grouping -CO-O-CO-, or are each -COOH or -COO-alkyl having 1-6 C atoms in the alkyl.

10. A polymer according to claim 8 or 9, which contains recurring structural elements of the formulae XI, XIII to XV or XVII to XX and in particular recurring structural elements of the formulae XI, XIV or XVII, and also, if desired, identical or different recurring structural elements of the formula XXI, in which formulae R'' is hydrogen or methyl, $Q_1$ is -O-, $Q_2$ and $Q_3$ independently of one another are -O- or -NH-, p is the number 1, n is the number 1 or 2, Y is $-OR_1-$ or $-NHR_1-$, $R_1$ is alkylene having 2-6 C atoms or, if $Q_2$ is -O-, also $-CH_2CH_2OCH_2CH_2-$ or $-(CH_2CH_2O)_2-CH_2-CH_2-$, X and X' are hydrogen, Z and $Z_1$ are hydrogen or methyl or methoxy bonded in the 7-position, $X_5$ is hydrogen, $X_4$ is hydrogen or methyl and $X_6$ is hydrogen, $-OCOCH_3$, -COOH or -COO-alkyl having 1-8 C atoms in the alkyl, or $X_4$ and $X_5$ are each hydrogen

and $X_6$ is -CN, chlorine, phenyl or alkoxy having 1-6 C atoms, or, if $X_4$ is hydrogen, $X_5$ and $X_6$ together are also -CO-O-CO-, and the groupings $-CO-(Y)_{n-1}$ are bonded in the 1-position or 3-position to the benzene ring of the radical «Th» or «$Th_1$».

11. A process for the preparation of a polymer according to claim 6, which comprises either

A) reacting a compound of the formula Ie

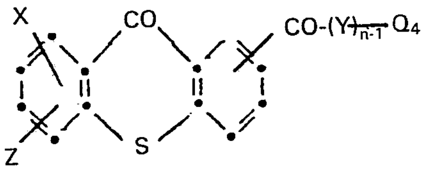

(Ie)

in which $Q_4$ is $-OCH=CH_2$, $-OCH_2CH=CH_2$, $-SCH_2CH=CH_2$ or $-NHCH_2CH=CH_2$ if n is 1 and is $-OCO-C(R'')=CH_2$, $-SCO-C(R'')=CH_2$, $-NHCO-C(R'')=CH_2$, $-OCH=CH_2$, or -N(CO)(CO) if n is 2, or is also $-CH=CH_2$ if $R_1$ is alkylene or phenylene, and X, Y, Z and n are as defined under formula I', if desired in the presence of comonomers, the molar ratio of the compound of the formula Ie to the comonomers being 1:49 to 1:0, or

B) reacting a compound of the formula If

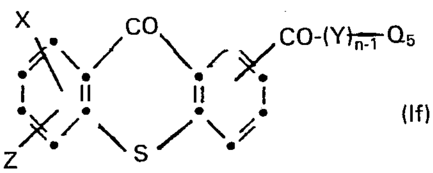

in which X' is hydrogen, halogen, -CN, $-NO_2$, phenylsulfonyl or alkylsulfonyl, alkyl, alkoxy, alkylthio, N,N-dialkylamino or -CO-alkyl having, in each case, 1-4 C atoms in the alkyl moieties, $Z_1$ is hydrogen, halogen or alkyl, alkoxy, alkylthio or N,N-dialkylamino having, in each case 1-4 C atoms in the alkyl moieties and $Q_5$ is $-OCH_2CH—CH_2$ (O) or $-OCH_2-COOH$ if n is 1, and is -OH, -SH, $-NH_2$, -NHR', $-SO_3H$, -COOH, -COCl, -NCO or $-OCH_2CH—CH_2$ (O) if n is 2, and Y and n are as defined under formula I', and R' is alkyl having 1 to 5 carbon atoms, or a salt of a compound of the formula If, in which $Q_5$ is $-NH_2$ or -NHR', with a polymer containing corresponding functional groups, in a ratio of 1:50 to 1:1, based on the number of recurring structural elements in the polymer.

12. The use of a compound of the formula I according to claim 1 or of a polymer according to claim 6 as a sensitiser for photocrosslinkable polymers.

13. The use of a compound of the formula I according to claim 1 or of a polymer according to claim 6 as an initiator for the photopolymerisation of ethylenically unsaturated compounds or for the photochemical crosslinking of polyolefines.

**Revendications**

1. Composés répondant à la formule I

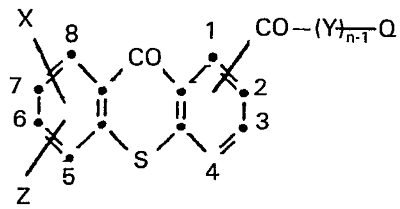

dans laquelle

n représente le nombre 1 ou le nombre 2, X représente l'hydrogène, un halogène, -CN, -OH, -SH, $-NO_2$, $-NH_2$, un phénylsulfonyle, un alkylsulfonyle, un alkyle, un alcoxy, un alkylthio, un N,N-dialkylamino ou un alkylcarbonyle, qui contiennent chacun de 1 à 4 atomes de carbone dans le parties alkyles, Z représente l'hydrogène, un halogène, -OH, -SH, un alkyle, un alcoxy, un alkylthio ou un N,N-dialkylamino contenant chacun de 1 à 4 atomes de carbone dans les parties alkyles, Y représente un radical $-OR_1-$, $-SR_1-$ ou $-N(R_2)R_1-$, où $R_1$ représente un alkylène, éventuellement ramifié, contenant de 2 à 23 atomes de carbone au total et de 2 à 13 atomes de carbone dans la chaîne principale, un cyclopentylène, un cyclohexylène, un phénylène, un radical répondant à l'une des formules

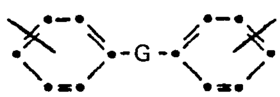 et 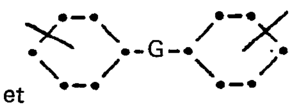

ou encore, lorsque n est égal à 2 et que Q représente -OH ou $-OCO-C(R'')=CH_2$, un radical $-(CH_2CH_2O)_x-CH_2CH_2-$, $R_2$ représente l'hydrogène ou un alkyle, éventuellement ramifié, contenant de 1 à 23 atomes de carbone au total et de 1 à 13 atomes de carbone dans la chaîne principale, G représente $-CH_2-$, $-CH_2CH_2-$, $-C(CH_2)_2-$, -O-, $-SO_2-$ ou -NH-, et x désigne un nombre entier de 1 à 5, Q représente, lorsque n est égal à 1, un radical $-OCH_2CH—CH_2$ (O), $-OCH_2-COOH$, $-OCH=CH_2$, $-OCH_2CH=CH_2$, $-SCH_2CH=CH_2$ ou $-NHCH_2CH=CH_2$ et, lorsque n est égal à 2, un radical -OH, -SH, $-NH_2$, -NHR', $-SO_3H$, -COOH, -COCl, -NCO, $-OCO-C(R'')=CH_2$, $-SCO-C(R'')=CH_2$, $-NHCO-C(R'')=CH_2$, $-OCH=CH_2$, $-OCH_2CH—CH_2$ (O), -N(CO)(CO), ou dans le cas où $R_1$ représente un alkylène ou un phénylène, un radical $-CH=CH_2$, R' représente un alkyle contenant de 1 à 5 atomes de carbone et R'' l'hydrogène ou un méthyle, ainsi que les sels que forment les composés de formule I dans lesquels Q représente $-NH_2$ ou -NHR'.

2. Composés de formule I selon la revendication 1, dans lesquels Z représente l'hydrogène, n, X, Y et Q ont les significations données et le groupement $-CO-(Y)_{n-1}$ Q se trouve en position 1 ou en position 3.

3. Composés de formule I selon la revendication 1, dans lesquels X représente l'hydrogène, Z représente l'hydrogène ou, en position 7, un méthyle ou un méthoxy, le radical $-CO-(Y)_{n-1}$ Q se trouve en position 1 ou en position 3, n est égal à 1 et dans ce cas Q représente $-OCH_2CH—CH_2$ (O) ou $-OCH=CH_2$,

ou n est égal à 2 et alors Y représente $-OR_1-$ ou $-NHR_1-$ (le symbole $R_1$ désignant un alkylène en $C_2$-$C_6$ ou, dans le cas où Q représente -OH ou $-OCO-C(R'')=CH_2$, également un radical $-CH_2CH_2OCH_2CH_2-$ ou $-(CH_2CH_2O)_2CH_2CH_2-$), et Q représente -OH, $-NH_2$, $-NHCH_3$, -COOH, -COCl, $-OCO-C(R'')=CH_2$, $-OCH=CH_2$, 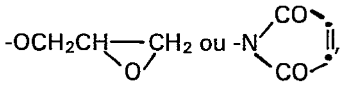

ainsi que les sels que forment, avec des acides minéraux, ceux des composés de formule I dans lesquels Q représente $-NH_2$ ou $-NHCH_3$.

4. Composés de formule I selon la revendication 3, dans lesquels Q représente, dans le cas où n est égal à 1, un radical $-OCH=CH_2$ et, dans le cas où n est égal à 2, un radical -OH, $-NH_2$, $-OCH=CH_2$ ou $-OCO-C(R'')=CH_2$, ainsi que les sels que forment avec HCl ceux des composés de formule I dans lesquels Q représente $-NH_2$.

5. Procédé de préparation de composés de formule I et de leurs sels conformes à la définition, selon la revendication 1, procédé caractérisé en ce que:

a) on fait réagir un composé répondant à la formule II

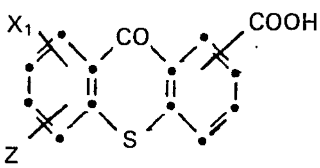

(II)

un ester alkylique en $C_1$-$C_6$ d'un composé de formule II ou un chlorure d'acide d'un composé de formule II dans lequel Z et $X_1$ ne représentent ni -SH ni -OH, avec un composé répondant à la formule IIa

$$H(Y)_{n-1}\,Q' \qquad (IIa)$$

ou, dans le cas où Q' représente $-NH_2$ ou -NHR', un sel d'un composé de formule IIIa, de manière à obtenir un composé de formule Ia

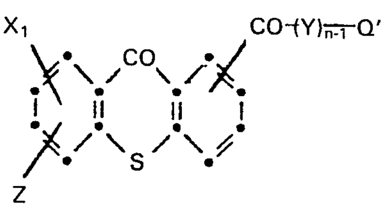

(Ia)

ou des sels correspondants,

b) on fait réagir un composé de formule II ou un ester alkylique en $C_1$-$C_6$ d'un tel composé avec un composé de formule IIIb

$$CH_2=CH-OCO-R' \qquad (IIIb)$$

de manière à obtenir un composé de formule Ib

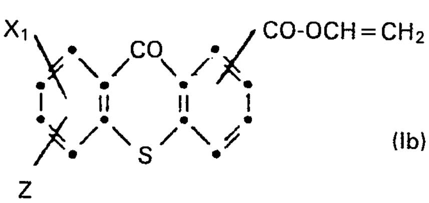

(Ib)

ou

c) on fait réagir un chlorure d'acide d'un composé de formule II d'abord avec un sel d'un composé de formule IIIc

$$H-Y-NH_2 \qquad (IIIc)$$

réaction qui conduit au sel correspondant d'un composé de formule IV

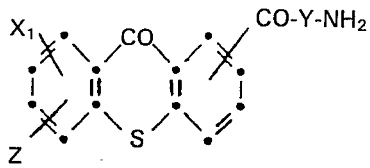

(IV)

et on fait réagir ce sel, en présence d'un solvant organique inerte, avec le phosgène, de manière à obtenir un composé de formule Ic

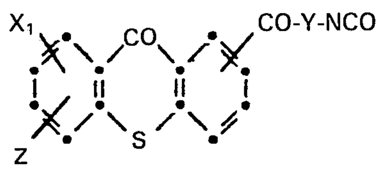

(Ic)

(dans les formules précédentes Y, Z, R' et n ont les significations qui ont été données à propos de la formule I, $X_1$ représente un atome d'hydrogène ou d'halogène, un radical -CN, -OH, -SH, $-NO_2$ ou phénylsulfonyle, ou un radical alkylsulfonyle, alkyle, alcoxy, alkylthio, N,N-dialkylamino ou alkylcarbonyle contenant de 1 à 4 atomes de carbone dans chacune des parties alkyles, Q' représente, lorsque n est égal à 1, un radical 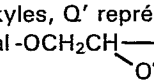, $-OCH_2-COOH$, $-OCH_2CH=CH_2$, $-SCH_2CH=CH_2$ ou $-NHCH_2CH=CH_2$ et, lorsque n est égal à 2, un radical -OH, -SH, $-NH_2$, -NHR', $-SO_3H$, -COOH, $-OCO-C(R'')=CH_2$, $-SCO-C(R'')=CH_2$, $-NHCO-C(R'')=CH_2$, 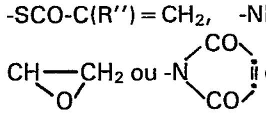 ou encore, lorsque $R_1$ représente un alkylène en un phénylène, un radical $-CH=CH_2$, et R'' représente l'hydrogène ou un méthyle), et ensuite, le cas échéant, on transforme le radical $X_1$ dans les composés de formule Ia, Ib ou Ic en un radical X ayant une autre signification, et/ou l'éventuel radical -COOHQ' dans la formule Ia, par traitement avec des agents de chloration appropriés, en un radical -COCl.

6. Polymère dont la masse moléculaire moyenne est d'au moins 1000 et qui contient des radicaux de thioxanthones latéraux répondant à la formule I'

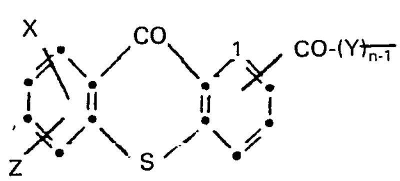

(I')

dans laquelle n désigne le nombre 1 ou le nombre 2, X représente l'hydrogène, un halogène, -CN, -OH,

-SH, $-NH_2$, $-NO_2$, un phénylsulfonyle ou un alkylsulfonyle, un alkyle, un alcoxy, un alkylthio, un N,N-dialkylamino ou un alkylcarbonyle qui contiennent chacun, dans leur partie alkyle ou dans chacune de leur partie alkyle, de 1 à 4 atomes de carbone, Z représente l'hydrogène, un halogène, -OH, -SH ou un alkyle, un alcoxy, un alkylthio ou un N,N-dialkylamino contenant chacun, dans leur partie alkyle ou dans chacune de leurs parties alkyles, de 1 à 4 atomes de carbone, Y représente $-OR_1-$, $-SR_1-$ ou $-N(R_2)R_1-$ où $R_1$ représente un alkylène éventuellement ramifié qui contient de 2 à 23 atomes de carbone au total et de 2 à 13 dans la chaîne principale, un cyclopentylène, un cyclohexylène, un phénylène,

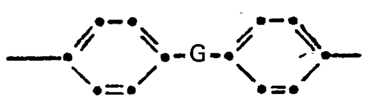 , 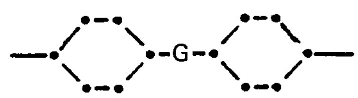

ou $-(CH_2CH_2O)_x-CH_2CH_2-$, $R_2$ représente l'hydrogène ou un alkyle, éventuellement ramifié, qui contient de 1 à 23 atomes de carbone au total et de 1 à 13 dans la chaîne principale, G représente $-CH_2-$, $-CH_2CH_2-$, $-C(CH_3)_2-$, -O-, $-SO_2-$ ou -NH- et x désigne un nombre entier de 1 à 5, la proportion de radicaux de formule I' étant d'au moins 2% relativement au nombre des unités structurales répétées du polymère.

7. Polymère selon la revendication 6 dans lequel la proportion de radicaux de formule I' est de 20 à 100% relativement au nombre des unités structurales répétées du polymère.

8. Polymère selon la revendication 6 qui a une masse moléculaire moyenne de 1000 à 100.000 et qui contient des unités structurales répétées répondant à l'une des formules XI à XX

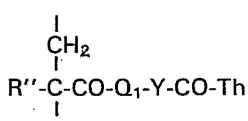 (XI)

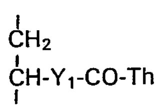 (XII)

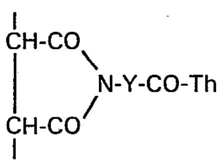 (XIII)

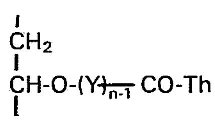 (XIV)

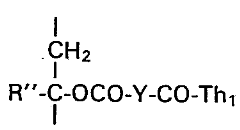 (XV)

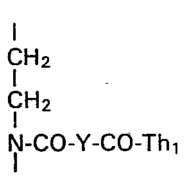 (XVI)

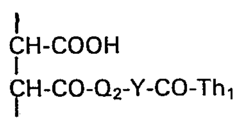 (XVII)

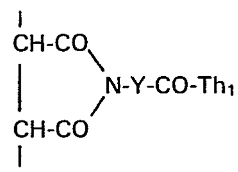 (XVIIa)

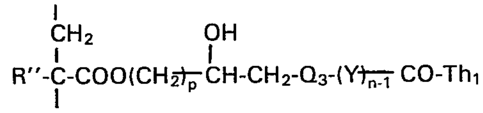 (XVIII)

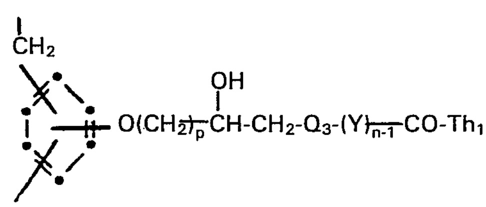 (XIX)

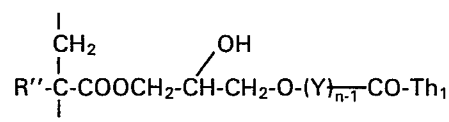 (XX)

dans lesquelles «Th» représente un radical de formule I''

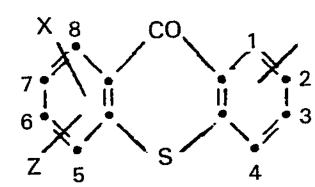 (I'')

et «$Th_1$» représente un radical de formule I'''

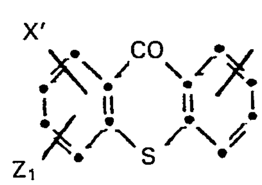 (I''')

X, Y, Z et n ont les significations qui ont été données plus haut à propos de la formule I', X' représente l'hydrogène, un halogène, -CN, $-NO_2$, un phénylsulfonyle ou un alkylsulfonyle, un alkyle, un alkoxy, un alkylthio, un N,N-dialkylamino ou un alkylcarbonyle contenant chacun, dans leur partie alkyle ou dans chacune des parties alkyles, de 1 à 4 atomes de carbone, $Z_1$ représente l'hydrogène, un halogène ou un alkyle, un alkoxy, un alkylthio ou un N,N-di-

alkylamino contenant chacun, dans sa partie alkyle ou dans chacune de ses parties alkyles de 1 à 4 atomes de carbone, $Q_1$ représente -O-, -S-, ou -NH-, $Q_2$ représente -O-, -S-, -NH- ou -NR'-, $Q_3$ représente -OCO-, -O-, -S-, -NH- ou -NR'-, R' représente un alkyle en $C_1$-$C_5$, R'' représente l'hydrogène ou un méthyle, p représente le nombre 1 ou le nombre 2, $Y_1$ représente $-OR_1-$, $-SR_1-$ ou $-NH-R_1-$, et $R_1$ représente un alkylène, éventuellement ramifié, qui contient de 2 à 23 atomes de carbone au total et de 2 à 13 dans sa chaîne principale, ou un phénylène.

9. Copolymère selon la revendication 8 qui est constitué d'unités structurales répétées répondant à l'une des formules XI à XX et d'unités structurales répétées, identiques ou différentes, qui répondent à la formule XXI

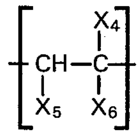

dans laquelle $X_5$ représente l'hydrogène, $X_4$ représente l'hydrogène, le chlore ou un méthyle et $X_6$ représente l'hydrogène, un méthyle, le chlore, -CN, -COOH, $-CONH_2$, un phényle, un méthylphényle, un méthoxyphényle, un cyclohexyle, un pyridyle, un imidazolyle, un pyrrolidyle, un alcoxycarbonyle contenant de 1 à 12 atomes de carbone dans sa partie alkyle, un phénoxycarbonyle, un glycidyloxycarbonyle, un hydroxyalcoxycarbonyle contenant de 1 à 3 atomes de carbone dans sa partie alkyle, un alcoxycarbonyle contenant de 1 à 4 atomes de carbone dans sa partie alkyle, un benzoyloxy, un alkylcarbonyle contenant de 1 à 3 atomes de carbone dans sa partie alkyle, un alcoxy en $C_1$-$C_{20}$ ou un phénoxy, ou encore $X_4$ représente l'hydrogène tandis que $X_5$ et $X_6$ forment ensemble un groupement -CO-O-CO- ou représentent chacun un radical -COOH ou un radical alcoxycarbonyle contenant de 1 à 6 atomes de carbone dans sa partie alkyle.

10. Polymère selon l'une des revendications 8 et 9 qui contient des unités structurales répétées répondant à l'une des formules XI, XIII à XV ou XVII à XX, et plus particulièrement des unités structurales répétées répondant aux formules XI, XIV ou XVII, et éventuellement aussi des unités structurales répétées, identiques ou différentes, répondant à la formule XXI, dans lesquelles R'' représente l'hydrogène ou un méthyle, $Q_1$ représente -O-, $Q_2$ et $Q_3$ représentent chacun, indépendamment l'un de l'autre, -O- ou -NH-, p représente le nombre 1, n le nombre 1 ou le nombre 2, Y représente $-OR_1-$ ou $-NHR_1-$, $R_1$ désignant un radical alkylène en $C_2$-$C_6$ ou, lorsque $Q_2$ est -O-, un radical $-CH_2CH_2OCH_2CH_2-$ ou $-(CH_2CH_2O)_2-CH_2CH_2-$, X ou X' représente l'hydrogène, Z ou $Z_1$ représente l'hydrogène ou, en position 7, un radical méthyle ou méthoxy, $X_5$ représente l'hydrogène, $X_4$ représente l'hydrogène ou un méthyle et $X_6$ représente l'hydrogène, $-OCOCH_3$, -COOH ou un alcoxycarbonyle contenant de 1 à 8 atomes de carbone dans sa partie alkyle, ou encore $X_4$ et $X_5$ représentent chacun l'hydrogène et $X_6$ représente -CN, le chlore, un phényle ou un alcoxy en $C_1$-$C_4$, ou $X_4$ représente l'hydrogène et $X_5$ et $X_6$ forment ensemble un radical -CO-O-CO-, et les groupements $-CO-(Y)_{n-1}-$ se trouvent en position 1 ou en position 3 sur le noyau benzénique du radical «Th» ou «$Th_1$».

11. Procédé de préparation d'un polymère selon la revendication 6, procédé caractérisé en ce que ou bien:

A) on fait réagir un composé répondant à la formule Ie

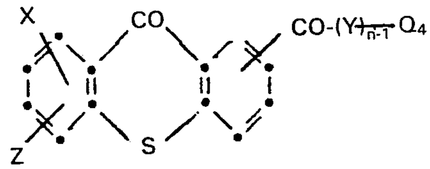

(Ie)

dans laquelle $Q_4$ représente, lorsque n est égal à 1, un radical $-OCH=CH_2$, $-OCH_2CH=CH_2$, $-SCH_2CH=CH_2$ ou $-NHCH_2CH=CH_2$, ou, lorsque n est égal à 2, un radical $-OCO-C(R'')=CH_2$, $-SCO-C(R'')=CH_2$, $-NHCO-C(R'')=CH_2$, $-OCH=CH_2$, 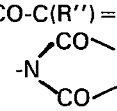 ou, dans le cas où $R_1$ est un alkylène ou un phénylène, également un radical $-CH=CH_2$, X, Y, Z et n ont les significations qui ont été données à propos de la formule I', éventuellement en présence de comonomères, le rapport molaire du composé de formule Ie au comonomère étant compris entre 1:49 et 1:0, ou bien:

B) on fait réagir un composé répondant à la formule If

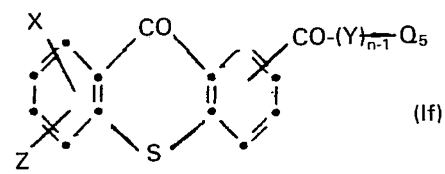

(If)

dans laquelle X' représente l'hydrogène, un halogène, -CN, $-NO_2$, un phénylsulfonyle ou un alkylsulfonyle, un alkyle, un alcoxy, un alkylthio, un N,N-dialkylamino ou un alkylcarbonyle contenant chacun, dans sa partie alkyle ou dans chacune de ses parties alkyles, de 1 à 4 atomes de carbone, $Z_1$ représente l'hydrogène, un halogène ou un alkyle, un alcoxy, un alkylthio ou un N,N-dialkylamino contenant chacun, dans sa partie alkyle ou dans chacune de ses parties alkyles, de 1 à 4 atomes de carbone, $Q_5$ représente, lorsque n est égal à 1, un radical 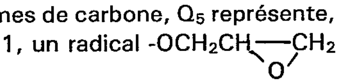 ou $-OCH_2-COOH$ et, lorsque n est égal à 2, un radical -OH, -SH, $-NH_2$, -NHR', $-SO_3H$, -COOH, -COCl, -NCO ou 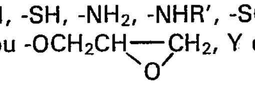, Y et n ont les significations données à propos de la formule I' et R' représente un alkyle en $C_1$-$C_5$, ou des sels de composés de formule If dans lesquels $Q_5$ représente $-NH_2$ ou -NHR', avec un polymère contenant des radicaux fonctionnels correspondants, dans un rapport molaire de 1:50 à 1:1 relativement au nombre des unités structurales répétées du polymère.

12. Application d'un composé de formule I selon

la revendication 1 ou d'un polymère selon la revendication 6 comme sensibilisateur pour des polymères photoréticulables.

13. Application d'un composé de formule I selon la revendication 1 ou d'un polymère selon la revendication 6 comme amorceur pour la photopolymérisation de composés éthyléniques ou pour la réticulation photochimique de polyoléfines.